(19) Europäisches Patentamt
European Patent Office
Office européen des brevets

(11) EP 3 936 547 B1

(12) EUROPEAN PATENT SPECIFICATION

(45) Date of publication and mention of the grant of the patent:
**28.08.2024 Bulletin 2024/35**

(21) Application number: **21184440.2**

(22) Date of filing: **08.07.2021**

(51) International Patent Classification (IPC):
***C08F 297/04*** *(2006.01)* ***C08F 8/04*** *(2006.01)*
***C08L 53/02*** *(2006.01)* ***C08L 91/00*** *(2006.01)*
***C08L 23/12*** *(2006.01)* ***C08J 5/18*** *(2006.01)*
*A61J 1/12 (2006.01)* *A61M 5/315 (2006.01)*
*F16J 15/00 (2006.01)* *C08K 3/36 (2006.01)*
*C08L 71/12 (2006.01)* *C08L 83/04 (2006.01)*

(52) Cooperative Patent Classification (CPC):
(C-Sets available)
**C08F 297/044; A61M 5/31513; C08F 8/04; C08J 5/18; C08L 53/025; F16J 15/102;**
C08J 2353/02; C08L 91/00 (Cont.)

# (54) HYDROGENATED BLOCK COPOLYMER, ELASTOMER COMPOSITION COMPRISING HYDROGENATED BLOCK COPOLYMER, SEAL MEMBER COMPRISING ELASTOMER COMPOSITION, PLUG BODY AND MEDICAL PLUG

HYDRIERTES BLOCKCOPOLYMER, ELASTOMERZUSAMMENSETZUNG MIT HYDRIERTEM BLOCKCOPOLYMER, DICHTUNGSELEMENT MIT ELASTOMERZUSAMMENSETZUNG, STOPFENKÖRPER UND MEDIZINISCHER STOPFEN

COPOLYMÈRE SÉQUENCÉ HYDROGÉNÉ, COMPOSITION ÉLASTOMÈRE COMPRENANT UN COPOLYMÈRE SÉQUENCÉ HYDROGÉNÉ, ÉLÉMENT D'ÉTANCHÉITÉ COMPRENANT UNE COMPOSITION ÉLASTOMÈRE, CORPS DE BOUCHON ET BOUCHON MÉDICAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.07.2020 JP 2020118630**
**06.07.2021 JP 2021112003**

(43) Date of publication of application:
**12.01.2022 Bulletin 2022/02**

(73) Proprietor: **Asahi Kasei Kabushiki Kaisha**
**Tokyo 100-0006 (JP)**

(72) Inventors:
- **Kameda, Ippei**
**Tokyo 100-0006 (JP)**
- **Shibuya, Kenta**
**Tokyo 100-0006 (JP)**
- **Hisasue, Takahiro**
**Tokyo 100-0006 (JP)**
- **Horiuchi, Mika**
**Tokyo 100-0006 (JP)**

(74) Representative: **Strehl Schübel-Hopf & Partner**
**Maximilianstrasse 54**
**80538 München (DE)**

(56) References cited:
**JP-A- 2020 019 947**
**US-A1- 2003 225 209**
**US-A1- 2007 112 102**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C08L 53/025, C08L 53/025, C08L 53/025;**
**C08L 53/025, C08L 91/00, C08L 91/00,**
**C08L 23/12, C08L 71/12, C08K 3/36, C08L 83/04;**
C08L 91/00, C08L 53/025, C08L 91/00, C08L 23/12,
C08L 71/12, C08K 3/36, C08L 83/04

## Description

### Technical Field

The present invention relates to a hydrogenated block copolymer, an elastomer composition comprising the hydrogenated block copolymer, and a seal member, a plug body, and a medical plug comprising the elastomer composition.

### Background Art

Block copolymers comprising a vinyl aromatic monomer unit and a conjugated diene monomer unit, which have elasticity comparable to that of vulcanized natural rubber and synthetic rubber at normal temperature, even when not vulcanized, and furthermore have fabricability comparable to that of a thermoplastic resin at high temperatures, are widely used in fields such as footwear, plastic modification, asphalt modification, and viscous adhesive materials, household products, packaging materials for consumer electrical appliances and industrial parts, toys, and the like. The hydrogenated products of the block copolymer (hydrogenated block copolymer), which has excellent weatherability and heat resistance, are widely used also in automotive parts and medical devices in addition to the application fields as described above.

Particularly, a hydrogenated block copolymer having a high molecular weight, which is excellent in mechanical properties in addition to the various characteristics described above, is combined with a liquid softener such as oil, a plasticizer, and a thermoplastic resin such as polypropylene to be used as a material for various industrial parts such as electric wire cables, automotive parts, medical plugs for medical use, syringe gaskets, and the like.

As polymers for use in these applications, a triblock SEBS (styrene - ethylene butylene - styrene) linear polymer (molecular weight in terms of polystyrene: of the order of about 200,000 to 500,000) is widely used because its high heat resistance and high mechanical strength are particularly excellent.

For example, Japanese Patent No. 5591346 discloses obtaining a product of a SEES polymer having a main peak molecular weight of 200,000 to 600,000 in a crumb form.

For example, Japanese Patent No. 2764746 discloses SEES having a number average molecular weight of 50,000 to 600,000.

Japanese Patent Laid-Open No. 2001-240720 discloses a composition containing a SEBS structure having a number average molecular weight of 250,000 or more.

Japanese Patent No. 3378582 discloses a coupled-type SEES polymer having a molecular weight of the coupling portion of substantially 15,000 to 300,000.

Japanese Patent No. 5324426 discloses a coupled-type and radial-structured SEES polymer having a peak molecular weight of a diblock portion of 230,000 to 275,000.

Japanese Patent No. 5703990 discloses a rubber plug body for medical use molded from a resin composition containing a hydrogenated block copolymer, a softener for hydrocarbon rubber, and a polyolefinic resin.

International Publication No. WO2018/139122 discloses a composition with an excellent balance among resealability, coring resistance, and needlestick resistance.

Japanese Patent Laid-Open No. 2020-19947 discloses a composition containing a special surface-treated silica blended, with an excellent balance among resealability, coring resistance, and needlestick resistance.

### Summary of Invention

### Problems to be Solved by Invention

A triblock SEBS linear polymer, however, has an excessively high viscosity and has problems of occurrence of an unmelted residue on compound processing associated with degradation in mixability with another resin, that is, occurrence of grains, occurrence of appearance defects on injection molding, insufficient film formability, and the like.

Having a high viscosity is not preferable also in the production stage of SEES.

Specifically, first, in a polymerization step, when a triblock SEBS linear polymer having a molecular weight of 250,000 or more is produced by common living anion polymerization, a trace amount of polymerization initiator is used. The molecular weight is markedly deviated due to the influence of a trace amount of a deactivation component (impurity) in the solvent or the monomer, and thus, the yield decreases. In order to avoid those described above, it is necessary to improve purification of the solvent and monomer, but such an improvement is economically disadvantageous. Further, in a hydrogenation reaction step, it is deemed that, when the diffusion efficiency of hydrogen in the system decreases, for example, an extremely long time is required to obtain an intended degree of hydrogenation, and the productivity declines. In a common solvent removal step comprising a steam stripping step and a dewatering extrusion step, events

may occur such as breakage of driving equipment due to an overload on the equipment, cleavage of the molecule chains of the polymer, and, in some cases, ignition. SEBS after dried, which may be often in a powder form, adheres to inside the step, resulting in decrease in the yield or contamination.

Japanese Patent No. 5591346 has successfully provided a product in a crumb form of a SEBS polymer having a main peak molecular weight of 200,000 to 600,000 but indicates that the production method is not easy, for example, the finishing step requires to be devised.

Particularly, each problem on processing/molding described above is required to be preferentially solved, and thus, measures have been taken such as addition of oil or increase in the amount of oil to be added in order to improve the compounding and molding processability. However, it is difficult to maintain heat resistance and mechanical strength, and the measures are insufficient from the viewpoint of the balance between compounding and molding processability with high heat resistance and high mechanical strength and also insufficient from the viewpoint of reduction in grains. Use of coupled type, not linear, high molecular weight SEBS is considered suitable in order to solve these problems, but the use thereof has not been sufficiently investigated.

In Japanese Patent No. 2764746, in respect of SEBS having a number average molecular weight of 50,000 to 600,000, a coupled type is also described in Description, but the SEBS is not described at all in Examples, and the number average molecular weight is the order of 300,000 at the maximum.

In Japanese Patent Laid-Open No. 2001-240720, in respect of a composition containing a SEBS structure having a number average molecular weight of 250,000 or more, a coupled type is also described in Description, but there is no mention in Examples, as in Japanese Patent No. 2764746, and the largest SEBS structure has a number average molecular weight of 280,000.

In Japanese Patent No. 3378582, in a coupled-type SEBS polymer, the coupling portion is limited to the linear type, and no radial structure is mentioned at all.

In Japanese Patent No. 5324426, in a coupled-type and radial-structured SEBS polymer, the diblock content is extremely small. In Example, the amount of a tri- or higher-branched component of a radial structure is also small. Thus, a structure mainly based on the radial structure is not optimized.

Accordingly, in a SEBS polymer of a conventional coupled type, particularly, a SEBS polymer mainly based on a radial structure, when a composition is cause to have processability and heat resistance (low compression set), room for improvement still remains in respect of a balance with a small amount of grains.

In medical plug applications, a SEBS polymer is required to have an excellent balance among the compounding and molding processability described above, high heat resistance and high mechanical strength, and the small amount of grains, and also required to have an excellent balance among resealability, coring resistance, needlestick resistance, oil bleed resistance, and a low odor property.

In Japanese Patent No. 5703990, a sealability test is conducted using a sealed PET bottle under conditions of a small amount of liquid, and sealability in use of a bag-shaped container has not been verified. Under atmospheric conditions where a container with a plug body has an air hole bored therein and has a large amount of a liquid therein, only an insufficient characteristic has been yet obtained on the resealability in the case where a needle is stuck in the plug body for a long time.

The composition described in International Publication No. WO2018/139122 is required to contain a polyphenylene ether resin and required to contain a relatively large amount of a non-aromatic softener blended thereto in order to adjust the hardness. Thus, there is a concern on oil bleeding or an odor derived from the polyphenylene ether resin.

The composition described in Japanese Patent Laid-Open No. 2020-19947 will have a high hardness and is thus required to have a relatively large amount of a non-aromatic softener blended thereto. Accordingly, in addition to the concern of oil bleeding, special silica is required, and thus, the composition is not versatile.

In view of the aforementioned problems of conventional art, an object of the present invention is thus to provide a hydrogenated block copolymer that has an excellent balance between processability and a low compression set property and, in use for a medical plug application, has an excellent balance among resealability, coring resistance, and needlestick resistance, and an elastomer composition containing the hydrogenated block copolymer.

Means for Solving Problems

In order to solve the above problems of conventional art, the inventors have conducted diligent research and, as a result, have found that a coupled-type hydrogenated block copolymer having a specific structure and an elastomer composition containing the hydrogenated block copolymer effectively solve the above problems, having accomplished the present invention.

That is to say, the present invention is as set forth below.

[1] A hydrogenated block copolymer (P) obtained by hydrogenation of a coupled polymer represented by the following formula (1):

(A-B)n-X (1)

wherein A represents a polymer block comprising a vinyl aromatic monomer unit as a main component, B represents a polymer block comprising a conjugated diene monomer unit as a main component, n is an integer of 1 or greater, and X represents a residue of a coupling agent or a residue of a polymerization initiator, wherein

a content of the vinyl aromatic monomer unit in the hydrogenated block copolymer (P) is 10 mass% to 40 mass%,
a peak top molecular weight of a diblock component corresponding to (A-B) in the formula (1) is 160,000 to 225,000,
a proportion of a dibranched component corresponding to a case in which n is 2 in the formula (1) is 10 mass% to 30 mass% based on the total hydrogenated block copolymer (P),
a proportion of a tribranched component corresponding to a case in which n is 3 in the formula (1) is 40 mass% to 70 mass% based on the total hydrogenated block copolymer (P), and
a ratio M (mass of tribranched component/mass of dibranched component) of the tribranched component to the dibranched component is 2 or more.

[2] The hydrogenated block copolymer according to [1], wherein the proportion of the diblock component is 10 mass% to 40 mass% based on the total hydrogenated block copolymer (P).
[3] The hydrogenated block copolymer according to [1] or [2], wherein a proportion of a tetra- or higher-branched component corresponding to a case in which n is an integer of 4 or more in the formula (1) is 10 mass% or less based on the total hydrogenated block copolymer (P).
[4] The hydrogenated block copolymer according to any of [1] to [3], wherein a weight average molecular weight of the total hydrogenated block copolymer (P) is 350,000 to 500,000.
[5] The hydrogenated block copolymer according to any of [1] to [4], wherein a proportion of a 1,2-bond and a 3,4-bond in the conjugated diene monomer unit in the hydrogenated block copolymer (P) is 50 mol% to 80 mol%.
[6] The hydrogenated block copolymer according to any of [1] to [5], wherein a degree of hydrogenation of the conjugated diene monomer unit in the hydrogenated block copolymer (P) is 80 mol% or more.
[7] An elastomer composition comprising the hydrogenated block copolymer according to any of [1] to [6].
[8] The elastomer composition according to [7], comprising 10 to 50 parts by mass of a polypropylene resin and 30 to 200 parts by mass of a non-aromatic softener based on 100 parts by mass of the hydrogenated block copolymer.
[9] The elastomer composition according to [7] or [8], wherein, when an elastomer composition sheet produced under the following conditions is cut into a 10 cm × 10 cm square and both the surfaces are observed with a microscope, the number of grains having a diameter of 200 μm or more calculated by the following expression is 0; [Production of Elastomer Composition Sheet]
a sheet material comprising the hydrogenated block copolymer is melt-kneaded with a twin screw extruder at a setting temperature of 230°C, and the obtained kneaded product is molded using a T die at a number of revolutions of the screws of 300 rpm to produce an elastomer composition sheet having a thickness of 500 μm;

Number of grains = [total number of grains having a diameter of 200 μm or more observed] × Y/X

wherein X represents a weight of the composition sheet (g), Y represents an amount of the hydrogenated block copolymer in the composition sheet (g), and a calculated value rounded off to the nearest whole number is employed as the number of the grains.
[10] The elastomer composition according to any of [7] to [9], comprising no polyphenylene ether resin.
[11] The elastomer composition according to any of [7] to [10], comprising no surface-treated silica.
[12] The elastomer composition according to any of [7] to [9], comprising no polyphenylene ether resin and surface-treated silica.
[13] A seal member comprising the elastomer composition according to any of [7] to [12].
[14] A plug body comprising the elastomer composition according to any of [7] to [12].
[15] A medical plug for use in a medical application, comprising the elastomer composition according to any of [7] to [12].

Advantages of Invention

The hydrogenated block copolymer of the present invention has an excellent balance between processability and a low compression set property and, in use for a medical plug application, has an excellent balance among resealability, coring resistance, and needlestick resistance.

Brief Description of Drawings

FIG. 1 shows schematic views of an exemplary plug body; and
FIG. 2 shows schematic views of an exemplary jig.

Mode for Carrying Out Invention

Hereinafter, an embodiment for carrying out the present invention (hereinafter referred to as "the present embodiment") will now be described in detail.

[Hydrogenated Block Copolymer]

A hydrogenated block copolymer of the present embodiment is a hydrogenated block copolymer (P) obtained by hydrogenation of a coupled polymer represented by the following formula (1):

$$(A\text{-}B)n\text{-}X \qquad (1)$$

wherein A represents a polymer block comprising a vinyl aromatic monomer unit as a main component, B represents a polymer block comprising a conjugated diene monomer unit as a main component, n is an integer of 1 or greater, and X represents a residue of a coupling agent or a residue of a polymerization initiator, wherein

the content of the vinyl aromatic monomer unit in the hydrogenated block copolymer (P) is 10 mass% to 40 mass%,
the peak top molecular weight of a diblock component corresponding to (A-B) in the formula (1) is 160,000 to 225,000,
the proportion of a dibranched component corresponding to a case in which n is 2 in the formula (1) is 10 mass% to 30 mass% based on the total hydrogenated block copolymer (P),
the proportion of a tribranched component corresponding to a case in which n is 3 in the formula (1) is 40 mass% to 70 mass% based on the total hydrogenated block copolymer (P), and
the ratio of the tribranched component to the dibranched component M (mass of tribranched component/mass of dibranched component) is 2 or more.

Conventionally, when the viscosity is lowered in order to improve the processability, mechanical properties such as a low compression set property usually decrease. However, the hydrogenated block copolymer of the present embodiment is caused to have a configuration as described above and thus can improve both properties: processability and a low compression set property in a well-balanced manner.
In the present embodiment, each monomer unit constituting the block copolymer is named after the monomer from which the monomer unit is derived.
For example, the "vinyl aromatic monomer unit" means a constitutional unit of a polymer produced as a result of polymerizing a monomer vinyl aromatic compound. The vinyl aromatic monomer unit is bonded to other monomer units via the vinyl groups of the vinyl aromatic compound.
Moreover, the "conjugated diene monomer unit" means a constitutional unit of a polymer produced as a result of polymerizing a monomer conjugated diene compound. The conjugated diene monomer unit is bonded to other monomer units via one of the two double bonds of the conjugated diene compound (1,2-bond or 3,4-bond) or bonded to other monomer units via both the two double bonds of the conjugated diene compound (1,4-bond).
The hydrogenated block copolymer (P) of the present embodiment comprises a polymer block A comprising a vinyl aromatic monomer unit as a main component (hereinafter, also simply referred to as "polymer block A") and a polymer block B comprising a conjugated diene monomer unit as a main component (hereinafter, also simply referred to as "polymer block B).
Here, the phrase "comprising a vinyl aromatic monomer unit as a main component" means that the amount of the vinyl aromatic monomer unit contained in the polymer block A is 75 mass% or more, preferably 80 mass% or more, and more preferably 90 mass% or more based on the polymer block A. The phrase "comprising a conjugated diene monomer unit as a main component" means that the amount of the conjugated diene monomer unit contained in the polymer block B is 90 mass% or more, preferably 96 mass% or more, and more preferably 99 mass% or more based on the polymer block B.
Examples of a "vinyl aromatic compound" constituting the "vinyl aromatic monomer unit" include, but are not limited to, styrene, α-methylstyrene, p-methylstyrene, divinylbenzene, 1,1-diphenylethylene, N,N-dimethyl-p-aminoethylstyrene, and N,N-diethyl-p-aminoethylstyrene. Of these, styrene, α-methylstyrene and p-methylstyrene are preferred

from the viewpoint of availability and productivity. Of these, styrene is particularly preferred. Only one of these may be used, or two or more of these may be used in combination.

The "conjugated diene compound" constituting the "conjugated diene monomer unit" is a diolefin having a pair of conjugated double bonds. Examples of the conjugated diene compound include, but are not limited to, 1,3-butadiene, 2-methyl-1,3-butadiene (isoprene), 2,3-dimethyl-1,3-butadiene, 1,3-pentadiene, 2-methyl-1,3-pentadiene, 1,3-hexadiene, and Farnesene. Examples of preferred diolefins include 1,3-butadiene and isoprene. Only one of these may be used, or two or more of these may be used in combination.

(Structure)

The structure of the hydrogenated block copolymer (P) of the present embodiment is a structure obtained by hydrogenation of a coupled polymer represented by the following formula (1):

$$(A\text{-}B)n\text{-}X \qquad (1)$$

wherein A represents a polymer block comprising a vinyl aromatic monomer unit as a main component, B represents a polymer block comprising a conjugated diene monomer unit as a main component, n is an integer of 1 or greater, and X represents a residue of a coupling agent or a residue of a polymerization initiator.

The hydrogenated block copolymer (P) having such a structure can be obtained by, for example, polymerizing the polymer block A and the polymer block B in this order and hydrogenating the coupled polymer obtained by coupling these polymers.

In the hydrogenated block copolymer (P) of the present embodiment, the proportion of the dibranched component corresponding to a case in which n is 2 in the formula (1) is 10 mass% to 30 mass%, preferably 10 mass% to 20 mass%, and more preferably 12 mass% to 18 mass% based on the total hydrogenated block copolymer (P). In the hydrogenated block copolymer (P) of the present embodiment, the proportion of the tribranched component corresponding to a case in which n is 3 in the formula (1) is 40 mass% to 70 mass%, preferably 45 mass% to 65 mass%, and more preferably 50 mass% to 60 mass% based on the total hydrogenated block copolymer (P). In the hydrogenated block copolymer (P) of the present embodiment, the ratio of the tribranched component to the dibranched component (mass of tribranched component/mass of dibranched component) is 2 or more, preferably 2 to 7, and more preferably 2 to 6.

With the proportion of each component within the range described above, the hydrogenated block copolymer (P) of the present embodiment enables the molecular weight to increase while increase in the viscosity is suppressed, enables the compression set to be improved, and additionally tends to have an excellent balance among resealability, coring resistance, and needlestick resistance in the case where the copolymer is molded into a medical plug.

From the viewpoint of the balance of improvement of the compression set and increase in the viscosity, the hydrogenated block copolymer (P) preferably contains a larger amount of a tri- or higher-branched multi-branched structure and may contain a higher-branched structure, that is, a tetra- or higher-branched structure. From the viewpoint of the performance balance between increase in the viscosity and a compression set, the hydrogenated block copolymer (P) preferably contains a larger amount of a tribranched component. When the hydrogenated block copolymer (P) is a tetra- or higher-branched component, the compression set is slightly improved, but increase in the viscosity is large and the performance balance tends to deteriorate. Additionally, it may be difficult to stably control the degree of branching. When a hydrogenated block copolymer (P) of a tetra- or higher-branched component is molded into a medical plug, the coring resistance tends to markedly deteriorate. Thus, the hydrogenated block copolymer (P) preferably comprises a tribranched component as a main component. More preferably, the proportion of a tetra- or higher-branched component is preferably 10 mass% or less, more preferably 9 mass% or less, and even more preferably 8 mass% or less based on the total hydrogenated block copolymer (P). The lower limit of the proportion of a tetra- or higher-branched component is not particularly limited and is, for example, 1 mass% based on the total hydrogenated block copolymer (P). Meanwhile, a dibranched component, which is generated in the process in which the tribranched component is contained as the main component, is preferably reduced as far as possible. In order to reduce the proportion of the dibranched component, particularly reduce the proportion to less than 10 mass%, it is necessary to ensure control of the deactivation component (impurity) in the solvent or the monomer and control of the purity of a coupling agent described below, and such controls are economically inefficient. From the viewpoint of practicality and the good balance between viscosity and a compression set and from the viewpoint of the balance between resealability and needlestick resistance in the case where the copolymer is molded into a medical plug, the proportion of the tribranched component to the dibranched component (mass of tribranched component/mass of dibranched component) is 2 or more.

In the hydrogenated block copolymer (P) of the present embodiment, a diblock component corresponding to a case in which n is 1 in the formula (1), that is, corresponding to (A-B) (hereinafter, also simply referred to as "diblock component") is preferably contained at a content of 10 mass% to 40 mass%, more preferably contained at a content of 15 mass% to 35 mass%, and even more preferably contained at a content of 20 mass% to 30 mass% based on the

total hydrogenated block copolymer (P), from the viewpoint of the balance between a compression set and viscosity. Also when the copolymer is molded into a medical plug, with the amount of the diblock component within the above range, the copolymer is excellent in the balance between resealability and needlestick resistance. When the amount of the diblock component is equivalent or higher than the lower limit value, the needlestick resistance tends to be favorable, and when the amount of diblock component is equivalent or lower than the upper limit value, the resealability tends to be favorable.

The coupling agent is not particularly limited as long as the mass ratio of each of the branched components is achieved, and examples of the coupling agent include polyalkenyl coupling agents. Preferred polyalkenyl coupling agents are not particularly limited. Examples thereof include divinylbenzenes, and m-divinylbenzene is preferred. Examples of the coupling agent also include, but are not particularly limited to, tetraalkoxysilanes such as tetraethoxysilane and tetramethoxysilane, alkyltrialkoxysilanes such as methyltrimethoxysilane, dialkyldialkoxysilanes such as dimethyldimethoxysilane, carboxylic acid ester compounds such as ethyl benzoate and methyl benzoate, tetrafunctional halogenated alkanes such as carbon tetrachloride, carbon tetrabromide, and tetrachloroethane, and glycidyl aromatic epoxy compounds such as a diglycidyl ether derived from a reaction between bisphenol A and epichlorohydrin. Only one of these may be used, or two or more of these may be used in combination. In order to obtain an intended branched structure with a single coupling agent, tetramethoxysilane or tetraethoxysilane is preferably used.

The proportion of each of the branched components can be controlled, although depending on the type of coupling agent, with the amount of the coupling agent to be added with respect to the amount of the polymerization initiator to be added and the temperature during a coupling reaction. In the case where the terminal structure during coupling is a vinyl aromatic monomer, branching is suppressed, in comparison with the case where the terminal structure is a conjugate diene monomer, and the amount of dibranched component tends to increase. Thus, the terminal structure is preferably adjusted to be a conjugate diene monomer.

The proportion of each branched component can be determined by vertically partition of each of peaks obtained by gel permeation chromatography (GPC) (solvent: tetrahydrofuran, temperature: 40°C) at each inflection point between the peaks. Detailed measurement conditions are shown in Examples.

(Molecular weight)

The peak top molecular weight of the diblock component in the hydrogenated block copolymer (P) of the present embodiment is 160,000 to 225,000 and preferably 170,000 to 200,000. When the peak top molecular weight of the diblock component is within the range described above, excellent balance between a low compression set property and processability is likely to be obtained. When the peak top molecular weight of the diblock component is within the range described above, the copolymer is also excellent in the balance between resealability and needlestick resistance in the case where the copolymer is used as a medical plug. When the peak top molecular weight of the diblock component is equivalent to or higher than the lower limit value, the needlestick resistance tends to be favorable, and when the peak top molecular weight of the diblock component is equivalent to or lower than the upper limit value, the resealability tends to be favorable.

The weight average molecular weight of the total hydrogenated block copolymer (P) of the present embodiment is preferably 350,000 to 500,000, more preferably 370,000 to 470,000, and even more preferably 400,000 to 450,000. In the hydrogenated block copolymer (P) of the present embodiment, when the weight average molecular weight is equivalent to or higher than the lower limit value, the compression set is improved, and when the weight average molecular weight is equivalent to or lower than the upper limit value, the processability is improved due to decrease in the viscosity while an improvement in the compression set is maintained. When the weight average molecular weight of the total hydrogenated block copolymer (P) is within the range described above, the copolymer is also excellent in the balance between resealability and needlestick resistance in the case where the copolymer is used as a medical plug. When the weight average molecular weight of the total hydrogenated block copolymer (P) is equivalent to or higher than the lower limit value, the needlestick resistance tends to be favorable, and when the weight average molecular weight of the total hydrogenated block copolymer (P) is equivalent to or lower than the upper limit value, the resealability tends to be favorable.

The peak top molecular weight of the diblock component can be determined by obtaining a molecular weight corresponding to the top of the peak obtained by gel permeation chromatography (GPC) (solvent: tetrahydrofuran, temperature: 40°C) from a standard polystyrene calibration curve. The weight average molecular weight of the total hydrogenated block copolymer (P) also can be determined using the same equipment. Detailed measurement conditions are shown in Examples.

(Content of vinyl aromatic monomer unit)

The content of the vinyl aromatic monomer unit in the hydrogenated block copolymer (P) of the present embodiment is 10 mass% to 40 mass%, preferably 15 mass% to 35 mass%, and more preferably 20 mass% to 30 mass%. In the hydrogenated block copolymer (P) of the present embodiment, when the content of the vinyl aromatic monomer unit is equivalent to or higher than the lower limit value, the compression set is improved as well as the blocking of the hydrogenated block copolymer itself can be suppressed. Alternatively, in the hydrogenated block copolymer (P) of the present embodiment, the content of the vinyl aromatic monomer unit is equivalent to or lower than the upper limit value, the viscosity decreases as well as the compression set becomes favorable. When the content of the vinyl aromatic monomer unit in the hydrogenated block copolymer (P) is brought within the range described above, the copolymer is excellent in the balance among coring resistance, needlestick resistance, and oil bleed resistance in the case where the copolymer is molded into a medical plug. When the content of the vinyl aromatic monomer unit in the hydrogenated block copolymer (P) is equivalent to or higher than the lower limit value, the needlestick resistance is improved. When the content of the vinyl aromatic monomer unit in the hydrogenated block copolymer (P) is equivalent to or lower than the upper limit value, the needlestick resistance is favorable as well as the coring resistance tends to be improved, and the oil bleed resistance is also further improved.

The content of the vinyl aromatic monomer unit in the hydrogenated block copolymer (P) can be measured with an ultraviolet spectrophotometer as described in Examples below.

The content of the vinyl aromatic monomer unit in the hydrogenated copolymer composition can be controlled within the predetermined numeric range by adjusting the amount of the vinyl aromatic compound added in the polymerization step.

(Content of conjugate diene monomer unit)

The content of the conjugate diene monomer unit in the hydrogenated block copolymer (P) of the present embodiment is preferably 60 mass% to 90 mass%, more preferably 65 mass% to 85 mass%, and even more preferably 70 mass% to 80 mass%.

(Proportion of 1,2-bond and 3,4-bond in conjugate diene monomer unit)

In the hydrogenated block copolymer (P) of the present embodiment, the conjugated diene monomer unit before hydrogenation is incorporated via a binding mode of a 1,2-bond, 3,4-bond, or 1,4-bond in the copolymer. The proportion of the 1,2-bonds and 3,4-bonds in the conjugate diene monomer unit in the hydrogenated block copolymer (P) of the present embodiment, in other words, the total proportion of the conjugated diene monomer units incorporated via a binding mode of the 1,2-bonds or 3,4-bonds is preferably 50 mol% to 80 mol%, more preferably 55 mol% to 75 mol%, and even more preferably 60 mol% to 70 mol% based on the total conjugated diene monomer units incorporated via the binding mode of the 1,2-bonds, 3,4-bonds, or 1,4-bonds. When the proportion of the 1,2-bonds and 3,4-bonds in the conjugate diene monomer unit in the hydrogenated block copolymer (P) of the present embodiment is equivalent to or higher than the lower limit value, crystallinity after a hydrogenation reaction described below is suppressed, decrease in the viscosity can be maintained, and additionally, as a composition, moderately favorable compatibility with a polypropylene resin is exhibited. Thus, grains, as an unmelted residue of the hydrogenated block copolymer, are reduced as well as favorable oil bleed resistance is exhibited without excessive ejection of the oil in the composition by the polypropylene resin. Here "grains" refer to projections comprising the hydrogenated block copolymer as the main component, which appear on the smooth surface of a thermoplastic elastomer composition sheet when the sheet is produced in accordance with the method described in Example described below. The "grains" include grains of various sizes, but grains that pose a problem here are grains having a diameter of 200 μm or more in a thermoplastic elastomer composition sheet provided under production conditions described in Examples below. Grains having a diameter of 200 μm or more may cause deterioration in the resealability or coring resistance in the case where the copolymer is molded into a medical plug. The "diameter" here is defined as the maximum diameter length of the grains.

The proportion of the 1,2-bonds and 3,4-bonds in the conjugate diene monomer unit can be controlled by adjusting a Lewis base (e.g., an ether or an amine), an amount of the Lewis base to be used, and the polymerization temperature. When the proportion of the 1,2-bonds and 3,4-bonds in the conjugate diene monomer unit is 80 mol% or less, it is not necessary to adjust the polymerization temperature to a low temperature in order to obtain a hydrogenated block copolymer (P), which is economical in production.

In the present embodiment, the proportion of the 1,2-bonds and 3,4-bonds in the conjugate diene monomer unit can be measured by nuclear magnetic resonance spectrometry analysis (NMR) or the like. The proportion of the 1,2-bonds and 3,4-bonds may be measured in a state either before or after hydrogenation. Specifically, the proportion can be measured by the method described in Examples below.

(Hydrogenation ratio in conjugate diene monomer unit)

Double bonds of the conjugate diene monomer unit contained in the hydrogenated block copolymer (P) of the present embodiment are hydrogenated. The proportion of the hydrogenated double bonds of the conjugated diene monomer units (hereinafter, also denoted by "degree of hydrogenation in the conjugate diene monomer unit") is 80 mol% or more, preferably 85 mol% or more, and more preferably 90 mol% or more. In the hydrogenated block copolymer (P) of the present embodiment, when the degree of hydrogenation in the conjugate diene monomer unit is 80 mol% or more, the compression set tends to be favorable, and additionally, when the copolymer is prepared into a composition, moderately favorable compatibility with a polypropylene resin can be obtained. The upper limit of the degree of hydrogenation in the conjugate diene monomer unit is 100 mol%.

The degree of hydrogenation in the conjugate diene monomer unit can be controlled by adjusting the amount of catalyst and the amount of hydrogen to be fed during the hydrogenation reaction, for example. The hydrogenation reaction speed can be controlled by adjusting the amount of catalyst, the amount of hydrogen to be fed, the pressure and temperature and the like during hydrogenation, for example.

The degree of hydrogenation in the conjugate diene monomer unit can be measured by the method described in Examples below.

[Method for Producing Hydrogenated Block Copolymer (P)]

The hydrogenated block copolymer (P) of the present embodiment can be produced by carrying out polymerization in an organic solvent, for example, with an organic alkali metal compound as the polymerization initiator to obtain a copolymer and then subjecting the copolymer to hydrogenation reaction.

The polymerization form may be batch polymerization or continuous polymerization, or may be a combination thereof. From the viewpoint of obtaining a copolymer having a narrow molecular weight distribution, a batch polymerization method is preferred.

The polymerization temperature is generally 0°C to 150°C, preferably 20°C to 120°C, more preferably 40°C to 100°C, and even more preferably, 40°C to 80°C.

The polymerization time depends on the polymer intended, and is usually with 24 hours and preferably 0.1 hours to 10 hours. From the viewpoint of obtaining a copolymer having a narrow molecular weight distribution and high strength, the polymerization time is more preferably 0.5 hours to 3 hours.

The polymerization pressure, which is not particularly limited, is only required to be in a pressure range sufficient maintaining nitrogen and the solvent in a liquid phase.

The polymerization system preferably contains no impurities such as water, oxygen and carbon dioxide, which may deactivate the polymerization initiator and the living polymer.

Examples of the organic solvent include, but are not particularly limited to, aliphatic hydrocarbons, such as n-butane, isobutane, n-pentane, n-hexane, n-heptane, and n-octane; alicyclic hydrocarbons, such as cyclohexane, cycloheptane, and methylcyclopentane; and aromatic hydrocarbons, such as benzene, xylene, toluene, and ethylbenzene.

The organic alkali metal compound as the polymerization initiator is preferably an organic lithium compound.

As the organic lithium compound, which is not particularly limited, for example, organic monolithium compounds, organic dilithium compounds, and organic polylithium compounds can be used. Examples of the organic lithium compound include, but are not limited to, ethyllithium, n-propyllithium, isopropyllithium, n-butyllithium, sec-butyllithium, t-butyllithium, phenyllithium, hexametylenedilithium, butadienyllithium, and isopropenyldilithium. Of these, from the viewpoint of polymerization activity, n-butyllithium and sec-butyllithium are preferred.

The amount of the organic alkali metal compound used as the polymerization initiator depends on the molecular weight of the polymer intended, and is generally preferably in the range of 0.01 phm to 0.5 phm (parts by mass per 100 parts by mass of the monomer), more preferably in the range of 0.03 phm to 0.3 phm, and even more preferably in the range of 0.05 phm to 0.15 phm.

The total amount of the 1,2-bonds and 3,4-bonds of the conjugate diene monomer unit before hydrogenation of the hydrogenated block copolymer (P) can be controlled by using a Lewis base (e.g., an ether or an amine). The amount of the Lewis base used is adjusted in accordance with the proportion of the 1,2-bonds and 3,4-bonds intended. Alternatively, adding the Lewis base and a metal alkoxide described below separately under two or more conditions can produce polymers each having a different proportion of the 1,2-bonds and 3,4-bonds in a polymer comprising a conjugated diene monomer unit as a main component.

Examples of the Lewis base include, but are not limited to, ether compounds, etheric compounds having two or more oxygen atoms, and tertiary amine compounds.

Examples of the tertiary amine compound include, but are not limited to, pyridine, N,N,N',N'-tetramethylethylenediamine, tributylamine, tetramethylpropanediamine, 1,2-dipiperidinoethane, and bis[2-(N,N-dimethylamino)ethyl]ether. Only one of these may be used, or two or more of these may be used in combination.

Preferable tertiary amine compounds are compounds having two amines. Furthermore, of these, compounds having a structure showing symmetry in the molecule are more preferable, and N,N,N',N'-tetramethylethylenediamine, bis[2-(N,N-dimethylamino)ethyl]ether, and 1,2-dipiperidinoethane are even more preferable.

In the step of producing the hydrogenated block copolymer (P) of the present embodiment, polymerization may be performed under coexistence of the Lewis base, organic lithium compound, and alkali metal alkoxide aforementioned. The alkali metal alkoxide herein is a compound represented by the general formula MOR, wherein M is an alkali metal, and R is an alkyl group.

The alkali metal of the alkali metal alkoxide is preferably sodium or potassium from the viewpoint of a high proportion of the 1,2-bonds and 3,4-bonds, a narrow molecular weight distribution, and a high polymerization speed.

The alkali metal alkoxide is, but are not limited to, preferably a sodium alkoxide, lithium alkoxide, or potassium alkoxide having an alkyl group having 2 to 12 carbon atoms, more preferably a sodium alkoxide or potassium alkoxide having an alkyl group having 3 to 6 carbon atoms, and even more preferably sodium t-butoxide, sodium t-pentoxide, potassium t-butoxide, or potassium t-pentoxide. Of these, sodium alkoxides such as sodium t-butoxide and sodium t-pentoxide are still more preferable.

In the step of producing the hydrogenated block copolymer (P) of the present embodiment, when polymerization is performed under coexistence of a Lewis base, an organic lithium compound, and an alkali metal alkoxide, the components preferably coexist in the molar ratio of the Lewis base to the organic lithium compound (Lewis base/organic lithium compound) and the molar ratio of the alkali metal alkoxide to the organic lithium compound (alkali metal alkoxide /organic lithium compound) described below.

Lewis base/organic lithium compound: 0.2 to less than 3.0 Alkali metal alkoxide/organic lithium compound: 0.3 or less

The molar ratio of Lewis base/organic lithium compound in the polymerization step is more preferably 0.5 or more from the viewpoint of a high proportion of the 1,2-bonds and 3,4-bonds and a high polymerization speed and is preferably 2.5 or less and more preferably 0.8 or more and 2.0 or less from the viewpoint of a narrow molecular weight distribution and high hydrogenation activity.

The molar ratio of alkali metal alkoxide/organic lithium compound is more preferably 0.2 or less, even more preferably 0.1 or less, and still more preferably 0.08 or less from the viewpoint of a narrow molecular weight distribution and high hydrogenation activity.

Furthermore, the molar ratio of alkali metal alkoxide /Lewis base is more preferably 0.1 or less, even more preferably 0.08 or less, still more preferably 0.06 or less, still more preferably 0.05 or less from the viewpoint of achieving a narrow molecular weight distribution and obtaining high hydrogenation activity.

In the step of producing the hydrogenated block copolymer (P) of the present embodiment, the hydrogenation method is not particularly limited. For example, hydrogenating a copolymer obtained as described above by supplying hydrogen in the presence of a hydrogenation catalyst can provide a hydrogenated block copolymer in which the double bond residue of the conjugated diene monomer unit has been hydrogenated.

When the polymerization step and hydrogenation step have been conducted in an inert hydrocarbon solvent, for example, removal of the inert hydrocarbon solvent enables the hydrogenated block copolymer to be isolated. Specific examples of a method of removing the solvent include, but are not particularly limited to, steam stripping. Water-containing crumbs are obtained by steam stripping, and drying the water-containing crumbs can provide the hydrogenated block copolymer.

In steam stripping, a surfactant is preferably used as a crumbing agent. Examples of such surfactant include, but are not particularly limited to, anionic surfactants, cationic surfactants, and nonionic surfactants, as described above. These surfactants can be generally added at 0.1 ppm to 3000 ppm to water in a stripping zone. In addition to the surfactant, a water-soluble salt of metal such as Li, Na, Mg, Ca, Al, or Zn can be used as a dispersion aid for crumbs.

The concentration of the hydrogenated block copolymer (P) in a crumb form dispersed in water, which is obtained through the polymerization step of hydrogenated block copolymer (P) and the steam stripping, is generally 0.1 mass% to 20 mass% (proportion based on water in the stripping zone). Within this range, crumbs having a favorable particle size can be obtained without disruption on operation. These crumbs of the hydrogenated block copolymer (P) is dewatered to adjust the water content to 1 mass% to 30 mass%. Thereafter, the crumbs are preferably dried until the water content reaches 1 mass% or less.

In the dewatering step of the crumbs, dewatering may be performed with a compression water squeezer such as a roll, a Banbury dehydrator, or a screw extruder-type squeezing dehydrator. Alternatively, dewatering and drying may be simultaneously performed with a conveyor and a box-type hot-air dryer.

In the method for producing the hydrogenated block copolymer (P) of the present embodiment, a deashing step for removing metals derived from a polymerization initiator or the like may be employed as required. In the method for producing the hydrogenated block copolymer (P) of the present embodiment, a step of adding an antioxidant, a neutralizing agent, a surfactant, or the like may be further employed as required.

Examples of the antioxidant include, but are not particularly limited to, hindered phenolic compounds, phosphorus compounds, and sulfur compounds. Only one of these may be used, or two or more of these may be used in

combination. Specific examples of the hindered phenolic compounds include, but are not particularly limited to, 2,6-di-t-butyl-4-methylphenol, n-octadecyl-3-(4'-hydroxy-3',5'-di-t-butylphenyl)propionate, [octadecyl-3-(3,5-dibutyl-t-butyl-4-hydroxyphenyl)propionate], 2,2'-methylenebis(4-methyl-6-t-butylphenol), 2,2'-methylenebis(4-ethyl-6-t-butylphenol), 2,4-bis[(octylthio)methyl]-o-cresol, 2-t-butyl-6-(3-t-butyl-2-hydroxy-5-methylbenzyl)-4-methylphenyl acrylate, 2,4-di-t-amyl-6-[1-(3,5-di-t-amyl-2-hydroxyphenyl)ethyl]phenyl acrylate, and 2-[1-(2-hydroxy-3,5-di-tert-pentylphenyl)] acrylate. Specific examples of the phosphorus compounds and sulfur compounds, include, but are not particularly limited to, 3,3'-thiodipropionate, 2-mercaptobenzimidazole, 4,6-bis(octylthiomethyl)-o-cresol, thiodiethylenebis[3-(3,5-di-tert-butyl-4-hydroxyphenyl)propionate], dilauryl thiodipropionate, lauryl stearyl thiodipropionate, pentaerythritol-tetrakis(6-lauryl thiopropionate), tris(nonylphenyl)phosphite, and tris(2,4-di-t-butylphenyl)phosphite.

The amount of the antioxidant to be added is preferably 0.01 parts by mass to 1 parts by mass, more preferably 0.05 parts by mass to 0.5 parts by mass, and even more preferably 0.1 parts by mass to 0.4 parts by mass based on 100 parts by mass of the hydrogenated block copolymer.

Examples of the neutralizing agent include, but are not particularly limited to, various metal stearates, hydrotalcite, and benzoic acid.

Examples of the surfactant include, but are not particularly limited to, anionic surfactants, nonionic surfactants, and cationic surfactants. Examples of the anionic surfactant include, but are not particularly limited to, a fatty acid salt, an alkyl sulfate ester salt, and an alkyl aryl sulfonate salt. Examples of the nonionic surfactant include, but are not particularly limited to, polyoxyethylene alkyl ether and polyoxyethylene alkyl aryl ether. Further, examples of the cationic surfactant include, but are not particularly limited to, an alkyl amine salt and a quaternary ammonium salt.

In the hydrogenated block copolymer (P) of the present embodiment, an antiblocking agent can be blended as required, into crumbs thereof, for the purpose of preventing blocking.

Examples of the antiblocking agent include, but are not limited to, calcium stearate, magnesium stearate, zinc stearate, polyethylene, polypropylene, ethylene bisstearyl amide, talc, and amorphous silica.

The amount of the antiblocking agent to be blended is preferably 500 to 10,000 ppm based on the hydrogenated block copolymer (P), and more preferably 1,000 to 7,000 ppm based on the hydrogenated block copolymer (P). The antiblocking agent is preferably blended in a state of attaching to the crumb surface and may be contained to some extent in the inside of the crumbs.

The hydrogenated block copolymer of the present embodiment also encompasses those to which the additives described above are blended.

[Elastomer Composition]

The elastomer composition of the present embodiment contains the hydrogenated block copolymer (P) described above. The elastomer composition of the present embodiment has few grains because of containing the hydrogenated block copolymer (P) described above. The elastomer composition of the present embodiment can be suitably used particularly in seal members, among others, in medical plug applications.

In the elastomer composition of the present embodiment, when an elastomer composition sheet produced under the following conditions is cut into a 10 cm $\times$ 10 cm square and both the surfaces are observed with a microscope, the number of grains having a diameter of 200 $\mu$m or more calculated by the following expression is preferably 0. With the number of the grains within the range, the elastomer composition sheet has an excellent appearance, and additionally, when placed in medical plug applications, tends to be excellent in resealability and coring resistance.

[Production of Elastomer Composition Sheet]

A sheet material comprising the hydrogenated block copolymer is melt-kneaded with a twin screw extruder at a setting temperature of 230°C, and the obtained kneaded product is molded using a T die at a number of revolutions of the screws of 300 rpm to produce an elastomer composition sheet having a thickness of 500 $\mu$m.

$$\text{Number of grains} = [\text{total number of grains having a diameter of 200 } \mu\text{m or more observed}] \times Y/X$$

wherein X represents the weight of the composition sheet (g), Y represents the amount of the hydrogenated block copolymer in the composition sheet (g), and a calculated value rounded off to the nearest whole number is employed as the number of the grains.

The elastomer composition of the present embodiment may contain a polypropylene resin.

Examples of the polypropylene resin include, but are not limited to, propylene homopolymers, or block copolymers and random copolymers of propylene with an olefin other than propylene, preferably an $\alpha$-olefin having 2 to 20 carbon atoms, and blends thereof.

Examples of the $\alpha$-olefins having 2 to 20 carbon atoms include, but are not limited to, ethylene, 1-butene, 1-hexene, 4-methyl-1-pentene, 1-octene, and 1-decene. An α-olefin having 2 to 8 carbon atoms is preferred, and ethylene, 1-butene, 1-hexene, or 4-methyl-1-pentene is more preferred.

Only one of the polypropylene resins may be used, or two or more of these may be used in combination.

The melt flow rate (MFR) of the polypropylene resin determined under conditions involving a temperature of 230°C and a load of 2.16 kg is preferably 0.1 g/10 min to 50 g/10 min, more preferably 0.5 g/10 min to 45 g/10 min, and even more preferably 1.0 g/10 min to 40 g/10 min. When MFR falls within the range, molding processability tends to be further improved.

Examples of the method for producing the polypropylene resin include, but are not limited to, a production method which involves polymerizing the monomers mentioned above using a Ziegler-Natta-type catalyst containing a titanium-containing solid transition metal component and an organometallic component in combination.

Examples of the transition metal component for use in the Ziegler-Natta-type catalyst include, but are not limited to, solid components containing titanium, magnesium and halogen as essential components and an electron-donating compound as an optional component, or titanium trichloride. Examples of the organometallic component include, but are not limited to, aluminum compounds.

Examples of the polymerization method for producing the polypropylene resin include, but are not limited to, a slurry polymerization method, a vapor-phase polymerization method, a bulk polymerization method, a solution polymerization method, and a multi-stage polymerization method combining these methods.

In these polymerization methods, only propylene is polymerized in the case of obtaining a propylene homopolymer, and propylene and a monomer other than propylene are polymerized in the case of obtaining a copolymer.

In the elastomer composition of the present embodiment, the content of the polypropylene resin is preferably 10 parts by mass to 100 parts by mass, more preferably 13 parts by mass to 75 parts by mass, and even more preferably 15 parts by mass to 50 parts by mass based on 100 parts by mass of the hydrogenated block copolymer (P).

When the content of the polypropylene resin is 10 parts by mass or more, favorable fluidity is obtained in the elastomer composition of the present embodiment to thereby provide excellent molding processability. When the content of the polypropylene resin is 100 parts by mass or less, favorable rebound resilience and flexibility are obtained in the elastomer composition of the present embodiment. In the case where the elastomer composition is used as a medical plug, with a content of polypropylene resin of 10 parts by mass or more, excellent molding processability can be obtained. Additionally, grains derived from an unmelted residue of the hydrogenated block copolymer (P) are reduced, and thus, excellent resealability or coring resistance can be obtained. With a content of the polypropylene resin of 50 parts by mass or less, excellent needlestick resistance and resealability can be obtained.

The elastomer composition of the present embodiment may further comprise a non-aromatic softener.

The non-aromatic softener is not particularly limited as long as the non-aromatic softener does not exhibit aromaticity and is capable of softening the elastomer composition of the present embodiment. Examples thereof include paraffin oil, naphthene oil, paraffin wax, liquid paraffin, white mineral oil, and plant-derived softeners. Among them, paraffin oil, liquid paraffin, or white mineral oil is preferred from the viewpoint of the low-temperature characteristics, leak-out resistance, and the like of a plug body for medical containers comprising the elastomer composition of the present embodiment.

The kinematic viscosity at 40°C of the non-aromatic softener is preferably 500 $mm^2$/sec or less. The lower limit value of the kinematic viscosity at 40°C of the non-aromatic softener is not particularly limited and preferably 10 $mm^2$/sec or more.

When the kinematic viscosity at 40°C of the non-aromatic softener is 500 $mm^2$/sec or less, the fluidity of the elastomer composition of the present embodiment tends to be further improved, and the molding processability thereof tends to be further improved.

The kinematic viscosity of the non-aromatic softener can be measured using a glass capillary viscometer.

When containing the non-aromatic softener having a kinematic viscosity at 40°C that falls within the range described above, the elastomer composition of the present embodiment tends to have a favorable non-aromatic softener retention properties, that is, oil bleed resistance.

In the elastomer composition of the present embodiment, the content of the non-aromatic softener is preferably 75 parts by mass to 300 parts by mass, more preferably 85 parts by mass to 250 parts by mass, and even more preferably 90 parts by mass to 200 parts by mass based on 100 parts by mass of the hydrogenated block copolymer (P).

When the content of the non-aromatic softener is within the range, an excellent elastomer composition can be obtained due to the non-aromatic softener retention properties, and when in use as a medical plug, the elastomer composition tends to have excellent oil bleed resistance. When the elastomer composition is used in medical plug applications, in order to reduce risks such as growth of bacteria, a smaller amount of the non-aromatic softener used is preferred. When the hydrogenated block copolymer (P) of the present embodiment is used, use of a polyphenylene ether resin or inorganic filler described below can be eliminated or the amount thereof used can be reduced to thereby reduce the amount of the non-aromatic softener used.

Specifically, when no polyphenylene ether resin is used but an inorganic filler is used, the amount of the non-aromatic softener to be added is preferably 30 parts by mass to 250 parts by mass, more preferably 50 parts by mass to 200 parts by mass, and even more preferably 70 to 180 parts by mass based on 100 parts by mass of the hydrogenated block copolymer (P).

When no inorganic filler is used but a polyphenylene ether resin is used, the amount of the non-aromatic softener to be added is preferably 30 parts by mass to 250 parts by mass, more preferably 50 parts by mass to 230 parts by mass, and even more preferably 70 to 200 parts by mass based on 100 parts by mass of the hydrogenated block copolymer (P).

When neither polyphenylene ether resin nor inorganic filler is used, the amount of the non-aromatic softener to be added is preferably 20 parts by mass to 150 parts by mass, more preferably 30 to 120 parts by mass, and even more preferably 40 to 100 parts by mass based on 100 parts by mass of the hydrogenated block copolymer (P).

For the purpose of adjusting the balance among molding processability, resealability, needlestick resistance, and coring resistance, the amount of the polypropylene resin to be added can be adjusted. For example, when the amount of the non-aromatic softener to be added is small, increasing the polypropylene resin enables the fluidity, that is, processability to be adjusted. From the viewpoint of adjustment of the balance among molding processability, resealability, needlestick resistance, and coring resistance, the amount of the polypropylene resin to be added is preferably 10 parts by mass to 100 parts by mass, more preferably 13 parts by mass to 75 parts by mass, and even more preferably 15 parts by mass to 50 parts by mass based on 100 parts by mass of the hydrogenated block copolymer (P).

From the viewpoint described above, the elastomer composition of the present embodiment preferably comprises 10 to 50 parts by mass of the polypropylene resin and 30 to 200 parts by mass of the non-aromatic softener based on 100 parts by mass of the hydrogenated block copolymer.

The elastomer composition of the present embodiment may further contain an inorganic filler.

Examples of the inorganic filler include, but are not limited to, talc, calcium carbonate, calcium oxide, zinc carbonate, wollastonite, zeolite, wollastonite, silica, alumina, clay, titanium oxide, magnesium hydroxide, magnesium oxide, sodium silicate, calcium silicate, magnesium silicate, sodium aluminate, calcium aluminate, sodium aluminosilicate, zinc oxide, potassium titanate, hydrotalcite, barium sulfate, titanium black, and carbon black such as furnace black, thermal black, and acetylene black.

Only one of these inorganic fillers may be used, or two or more of these may be used in combination. Among these, talc, calcium carbonate, silica, or clay is preferred, and talc or calcium carbonate is more preferred, from the viewpoint of the resealability and the like of a medical plug comprising the elastomer composition of the present embodiment.

In the case where the elastomer composition is used as a material for a rubber plug for medical containers (medical plug), it is assumed that the medical plug is subjected to steam sterilization treatment at approximately from 110°C to 121°C in a state incorporated in a cap or a holder. In this case, a sufficient swaging effect might not be obtained if the dimension of the medical plug is changed by heating.

Use of a surface-treated inorganic filler can further improve the resealability after steam sterilization treatment of the medical plug for medical containers. When the hydrogenated block copolymer (P) of the present embodiment is used, no special surface treatment is required. Further, the amount of the inorganic filler to be added may be reduced, or addition of the inorganic filler itself may be eliminated. Then, the amount of the non-aromatic softener to be added for adjustment of the hardness also can be reduced.

Examples of the method for surface-treating the inorganic filler include a method involving bringing a surface treatment agent and/or a solution thereof into contact with the surface of the inorganic filler.

When the surface treatment agent is, for example, a fatty acid, a resin acid, a fat and oil, or a surfactant, the surface treatment is performed as dry treatment by mixing the surface treatment agent in a powder form with the inorganic filler, and melting and chemically reacting the mixture while crushing the mixture in a heated crusher, such as a ball mill or a roller mill, or a mixer, such as a ribbon blender or a Henschel mixer.

In the case of using a non-water-soluble surface treatment agent having a high melting point, the surface treatment is performed by preparing an emulsion of the surface treatment agent or a solution of the surface treatment agent dissolved in an alcohol, and stirring and mixing the emulsion or the solution with the inorganic filler while injecting the emulsion or the solution into a crusher or a mixer, followed by drying.

The inorganic filler is surface-treated in wet treatment by adding the surface treatment agent to slurry at the time of synthesis of the inorganic filler, and stirring the mixture in a mixer or the like with heating. In the case of using a surface treatment agent having a high melting point, the surface treatment is performed by using an emulsion of the surface treatment agent, and similarly adding the emulsion at the time of synthesis of the inorganic filler, followed by stirring.

In the case of using a water-soluble coupling agent as the surface treatment agent, the surface treatment is usually performed by pH-adjusting a mixed solution of water and ethanol, then adding the coupling agent into the solution, and spraying the mixture into a heated high-speed stirring mixer, such as a Henschel mixer, containing the inorganic filler to cause chemical reaction with the surface of the inorganic filler. In the case of using a non-water-soluble coupling

agent as the surface treatment agent, the inorganic filler is surface-treated by dissolving the coupling agent in acetone, an alcohol, or the like, and spraying the solution into a heated high-speed stirring mixer containing the inorganic filler in the same manner as above.

The surface treatment agent is typically a fatty acid, a resin acid, a fat and oil, a surfactant, or a coupling agent (e.g., silane, titanium, phosphoric acid, and carboxylic acid coupling agents), but is not limited thereto as long as the surface treatment agent can act on the surface of the inorganic filler.

Examples of the fatty acid include, but are not limited to: saturated fatty acids such as caproic acid, caprylic acid, pelargonic acid, capric acid, lauric acid, myristic acid, palmitic acid, stearic acid, and behenic acid, metal salts thereof, and modified products thereof; and unsaturated fatty acids such as oleic acid, linoleic acid, erucic acid, eicosadienoic acid, docosadienoic acid, linolenic acid, eicosatetraenoic acid, tetracosapentaenoic acid, and docosahexaenoic acid, metal salts thereof, and modified products thereof.

Examples of the resin acid include, but are not limited to, rosins having main components such as abietic acid, neoabietic acid, palustric acid, pimaric acid, isopimaric acid, and dehydroabietic acid, and derivatives thereof.

Examples of the fat and oil include, but are not limited to, soybean oil, linseed oil, coconut oil, and safflower oil.

Examples of the surfactant include, but are not limited to: fatty acid-type anionic surfactants such as sodium stearate and potassium stearate; sulfuric acid ester-type anionic surfactants such as polyoxyethylene alkyl ether sulfuric acid ester, long-chain alcohol sulfuric acid ester, and sodium salts and potassium salts thereof; sulfonic acid-type anionic surfactants such as alkylbenzenesulfonic acid, alkylnaphthalenesulfonic acid, paraffin sulfonic acid, α-olefin sulfonic acid, alkylsulfosuccinic acid, and sodium salts and potassium salts thereof; and nonionic surfactants such as polyethylene glycol, polyvinyl alcohol, and derivatives thereof.

Examples of the silane coupling agent include, but are not limited to: alkyl group-containing silane coupling agents such as dimethyldichlorosilane, trimethylchlorosilane, dimethyldimethoxysilane, trimethylmethoxysilane, dimethyldiethoxysilane, n-propyltrimethoxysilane, n-propyltriethoxysilane, hexyltrimethoxysilane, hexyltriethoxysilane, and polydimethylsiloxane; phenyl group-containing silane coupling agents such as phenyltrichlorosilane, phenyltrimethoxysilane, and phenyltriethoxysilane; vinyl group-containing silane coupling agents such as vinyltrimethoxysilane and vinyltriethoxysilane; styryl group-containing silane coupling agents such as p-styryltrimethoxysilane; epoxy group-containing silane coupling agents such as 3-glycidoxypropylmethyldimethoxysilane; methacryl group-containing silane coupling agents such as 3-methacryloxypropylmethyldimethoxysilane, acryl group-containing silane coupling agents such as 3-acryloxypropylmethyldimethoxysilane; and amino group-containing silane coupling agents such as hexamethyldisilazane and N-2-(aminoethyl)-3-aminopropylmethyldimethoxysilane.

Examples of the titanium coupling agent include, but are not limited to, titanium isostearate, tetrastearyl titanate, tetraisopropyl titanate, isopropyl triisostearoyl titanate, titanium octylene glycolate compounds, titanium diethanol aminate, titanium aminoethyl aminoethanolate, bis(dioctylpyrophosphate) oxyacetate titanate, tris(dioctylpyrophosphate) ethylene titanate, isopropyl dioctylpyrophosphate titanate, isopropyl tris(dioctylpyrophosphate) titanate, isopropyl tris(dodecylbenzenesulfonyl) titanate, titanium tetra-normal butoxide, titanium tetra-2-ethylhexoxide, tetraoctyl bis(ditridecylphosphite) titanate, tetraisopropyl bis(dioctylphosphite) titanate, and tetra(2,2-diallyloxymethyl-1-butyl)bis(ditridecyl)phosphite titanate.

Examples of the phosphoric acid coupling agent include, but are not limited to: phosphoric acid triesters such as tributyl phosphate, tripentyl phosphate, trihexyl phosphate, triheptyl phosphate, trioctyl phosphate, trinonyl phosphate, tridecyl phosphate, triundecyl phosphate, tridodecyl phosphate, tritridecyl phosphate, tritetradecyl phosphate, tripentadecyl phosphate, trihexadecyl phosphate, triheptadecyl phosphate, trioctadecyl phosphate, trioleyl phosphate, triphenyl phosphate, tricresyl phosphate, trixylenyl phosphate, cresyl diphenyl phosphate, and xylenyl diphenyl phosphate; acidic phosphoric acid esters such as monobutyl acid phosphate, monopentyl acid phosphate, monohexyl acid phosphate, monoheptyl acid phosphate, monooctyl acid phosphate, monononyl acid phosphate, monodecyl acid phosphate, monoundecyl acid phosphate, monododecyl acid phosphate, monotridecyl acid phosphate, monotetradecyl acid phosphate, monopentadecyl acid phosphate, monohexadecyl acid phosphate, monoheptadecyl acid phosphate, monooctadecyl acid phosphate, monooleyl acid phosphate, dibutyl acid phosphate, dipentyl acid phosphate, dihexyl acid phosphate, diheptyl acid phosphate, dioctyl acid phosphate, dinonyl acid phosphate, didecyl acid phosphate, diundecyl acid phosphate, didodecyl acid phosphate, ditridecyl acid phosphate, ditetradecyl acid phosphate, dipentadecyl acid phosphate, dihexadecyl acid phosphate, diheptadecyl acid phosphate, dioctadecyl acid phosphate, and dioleyl acid phosphate; thiophosphoric acid esters such as tributyl phosphorothionate, tripentyl phosphorothionate, trihexyl phosphorothionate, triheptyl phosphorothionate, trioctyl phosphorothionate, trinonyl phosphorothionate, tridecyl phosphorothionate, triundecyl phosphorothionate, tridodecyl phosphorothionate, tritridecyl phosphorothionate, tritetradecyl phosphorothionate, tripentadecyl phosphorothionate, trihexadecyl phosphorothionate, triheptadecyl phosphorothionate, trioctadecyl phosphorothionate, trioleyl phosphorothionate, triphenyl phosphorothionate, tricresyl phosphorothionate, trixylenyl phosphorothionate, cresyl diphenyl phosphorothionate, and xylenyl diphenyl phosphorothionate; chlorinated phosphoric acid esters such as tris-dichloropropyl phosphate, tris-chloroethyl phosphate, tris-chlorophenyl phosphate, and polyoxyalkylene-bis[di(chloroalkyl)]phosphate; and phosphorous acid esters such as dibutyl phosphite, dipentyl phosphite, dihexyl phosphite, diheptyl phosphite, dioctyl phosphite, dinonyl phosphite, didecyl phosphite, diundecyl phosphite, didodecyl phosphite, dioleyl phosphite, diphenyl phosphite, dicresyl phosphite, tributyl phosphite, tripentyl phosphite, trihexyl phosphite, triheptyl phosphite, trioctyl phosphite, trinonyl phosphite, tridecyl phosphite, triundecyl phosphite, tridodecyl phosphite, trioleyl phosphite, triphenyl phosphite, and tricresyl phosphite.

Examples of the carboxylic acid coupling agent include, but are not limited to, carboxylated polybutadiene and hydrogenated products thereof, carboxylated polyisoprene and hydrogenated products thereof, carboxylated polyolefin, carboxylated polystyrene, carboxylated styrene-butadiene copolymers and hydrogenated products thereof, and carboxylated nitrile rubber.

In the case of using the elastomer composition of the present embodiment as a material for a medical plug for medical containers, among these, a fatty acid or a modified product thereof, or a silane coupling agent is preferred as the surface treatment agent from the viewpoint of resealability after steam sterilization treatment. The silane coupling agent is more preferably a trimethylsilyl-based silane coupling agent or a dimethylsilyl-based silane coupling agent.

The combination with the inorganic filler is preferably calcium carbonate surface-treated with a fatty acid or a modified product thereof, or silica surface-treated with a silane coupling agent (hereinafter, also denoted by "surface-treated silica"), more preferably silica surface-treated with a silane coupling agent, and even more preferably silica surface-treated with a trimethylsilyl-based silane coupling agent or a dimethylsilyl-based silane coupling agent, from the viewpoint of resealability after steam sterilization treatment.

The elastomer composition of the present embodiment may not contain a surface-treated silica.

As described above, when the elastomer composition of the present embodiment contains the inorganic filler, the resealability can be improved in the case where the elastomer composition is used as a medical plug. When the hydrogenated block copolymer (P) of the present embodiment is used, the amount of the inorganic filler to be added may be reduced, or addition of the inorganic filler itself may be eliminated. Then, the amount of the non-aromatic softener to be added for adjustment of the hardness also can be reduced. In other words, the elastomer composition of the present embodiment may contain or may not contain the inorganic filler, and in applications in which higher resealability is required, the elastomer composition may contain an inorganic filler. The total content of the inorganic filler in such a case is preferably 10 parts by mass to 200 parts by mass, more preferably 50 parts by mass to 150 parts by mass, even more preferably 10 parts by mass to 100 parts by mass, and still more preferably 15 parts by mass to 90 parts by mass based on 100 parts by mass of the hydrogenated block copolymer (P).

When the content of the inorganic filler is within the range, in a medical plug for use in medical containers comprising the elastomer composition of the present embodiment, excellent resealability tends to be obtained.

The average primary particle size of the inorganic filler is preferably 0.01 $\mu$m to 5 $\mu$m, more preferably 0.01 $\mu$m to 4 $\mu$m, and even more preferably 0.01 $\mu$m to 3 $\mu$m.

When the average primary particle size of the inorganic filler is 0.01 $\mu$m or more, an effect of improving the flexibility of the elastomer composition may be obtained. When the average primary particle size of the inorganic filler is 5 $\mu$m or less, an effect of improving homogeneous dispersibility to the elastomer composition may be obtained.

The elastomer composition of the present embodiment may further contain a polyphenylene ether resin. When the polyphenylene ether resin is contained, the heat resistance, that is, the low compression set property can be improved. In the case where the elastomer composition is used as a medical plug, the resealability can be improved. When the hydrogenated block copolymer (P) of the present embodiment is used, the amount of the polyphenylene ether resin to be added may be reduced, or addition of the polyphenylene ether resin itself may be eliminated. Then, the amount of the non-aromatic softener to be added for adjustment of the hardness also can be reduced. It is also possible to reduce or eliminate the odor specific to the polyphenylene ether resin.

The elastomer composition of the present embodiment may not contain a polyphenylene ether resin and surface-treated silica.

The polyphenylene ether resin is preferably a homopolymer and/or copolymer having a repeating structure unit represented by the following general structure (I).

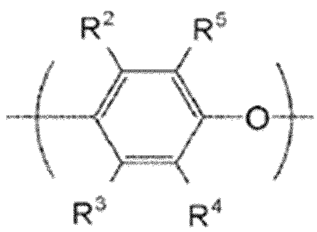

・・・(I)

Wherein, O represents an oxygen atom.

$R^2$ to $R^5$ each independently represent hydrogen, halogen, a primary or secondary $C_1$ to $C_7$ alkyl group, a phenyl group, a $C_1$ to $C_7$ haloalkyl group, a $C_1$ to $C_7$ aminoalkyl group, a $C_1$ to $C_7$ hydrocarbyloxy group, or a halohydrocarbyloxy group, where at least two carbon atoms separate the halogen and oxygen atoms.

A method for producing the polyphenylene ether resin is not particularly limited, and a known method can be employed. Examples thereof include the production methods and the like described in US Patent Nos. 3306874, 3306875, 3257357, and 3257358, Japanese Patent Laid-Open No. 50-51197, Japanese Patent Publication Nos. 52-17880 and 63-152628, and the like.

Examples of the polyphenylene ether resin include, but are not limited to, homopolymers such as poly(2,6-dimethyl-1,4-phenylene ether), poly(2-methyl-6-ethyl-1,4-phenylene ether), poly(2-methyl-6-phenyl-1,4-phenylene ether), and poly(2,6-dichloro-1,4-phenylene ether), and polyphenylene ether copolymers such as a copolymer of 2,6-dimethylphenol with other phenols (e.g., a copolymer with 2,3,6-trimethylphenol, and a copolymer with 2-methyl-6-butylphenol as described in Japanese Patent Publication No. 52-17880).

Among these, from the viewpoint of industrial productivity and temperature resistance performance, poly(2,6-dimethyl-1,4-phenylene ether), a copolymer of 2,6-dimethylphenol and 2,3,6-trimethylphenol, or a mixture thereof are preferred.

Further, the polyphenylene ether resin may be a wholly or a partly modified polyphenylene ether resin.

Here, the term "modified polyphenylene ether resin" refers to a polyphenylene ether resin which has been modified with at least one modifying compound which has in the molecular structure thereof at least one carbon-carbon double bond or triple bond, and has at least a carboxylic acid group, an acid anhydride group, an amino group, a hydroxyl group, or a glycidyl group.

Examples of the modifying compound which has in the molecular structure thereof at least one carbon-carbon double bond and a carboxylic acid group or an acid anhydride group include, but are not limited to, maleic acid, fumaric acid, chloromaleic acid, cis-4-cyclohexene-1,2-dicarboxylic acid, and acid anhydrides thereof.

Among these, from the viewpoint of the compatibility between the hydrogenated block copolymer (P) and the polyphenylene ether resin, fumaric acid, maleic acid, and maleic anhydride are preferred, and fumaric acid and maleic anhydride are more preferred.

Further, compounds in which at least one or two of the carboxyl groups of these unsaturated dicarboxylic acids have been esterified can also be used.

Examples of the modifying compound which has in the molecular structure thereof at least one carbon-carbon double bond and a glycidyl group include, but are not limited to, allylglycidyl ether, glycidyl acrylate, glycidyl methacrylate, and an epoxidized natural oil and fat. Among these, glycidyl acrylate and glycidyl methacrylate are preferred.

Examples of the modifying compound which has in the molecular structure thereof at least one carbon-carbon double bond and a hydroxyl group include, but are not limited to, an unsaturated alcohol represented by the general formula $C_nH_{2n-3}OH$, wherein n is a positive integer, and an unsaturated alcohol represented by the general formula $C_nH_{2n-5}OH$ or $C_nH_{2n-7}OH$, wherein n is a positive integer, such as allyl alcohol, 4-pentene-1-ol, and 1,4-pentadiene-3-ol.

One of the various modifying compounds described above may be used, or two or more of these may be used in combination.

The ratio of the modifying compound added in the modified polyphenylene ether resin is preferably 0.01 mass% to 5 mass% and more preferably 0.1 mass% to 3 mass%. In the modified polyphenylene ether resin, an unreacted portion of the modifying compound and/or a polymer formed from the modifying compound may remain in the amount of less than 1 mass%.

The reduced viscosity of the polyphenylene ether resin $\eta sp/C$ (0.5 g/dL, chloroform solution, measured at 30°C) is preferably in the range of 0.15 dL/g to 0.70 dL/g, more preferably in the range of 0.20 dL/g to 0.60 dL/g, and more preferably in the range of 0.25 dL/g to 0.50 dL/g.

When the reduced viscosity of the polyphenylene ether resin is 0.15 dL/g or more, in the elastomer composition of the present embodiment, a favorable compression set property tends to be obtained. When the reduced viscosity of the polyphenylene ether resin is 0.70 dL/g or less, the excellent processability tends to be obtained.

The reduced viscosity of the polyphenylene ether resin can be controlled to the numerical range described above by adjustment of the type of catalyst, polymerization period, and polymerization temperature in the production step of the polyphenylene ether resin.

In the present embodiment, two or more polyphenylene ether resins each having a different reduced viscosity may be blended and used wholly as a polyphenylene ether resin. In this case, the reduced viscosity of the mixture after mixing of a plurality of polyphenylene ether resins is preferably in the range of 0.15 dL/g to 0.70 dL/g, and the reduced viscosity of each of the polyphenylene ether resins may not be in the range of 0.15 dL/g to 0.70 dL/g.

The reduced viscosity of the polyphenylene ether resin can be measured by the method described in Examples below.

The number average molecular weight of the polyphenylene ether resin Mn is preferably 1,000 to 50,000, more preferably 1,500 to 50,000, and even more preferably 1,500 to 30,000. When the number average molecular weight of

the polyphenylene ether resin is within the range, a more excellent elastomer composition tends to be obtained via the compression set property.

The number average molecular weight of the polyphenylene ether resin can be determined using a calibration curve obtained by measurement on commercially available standard polystyrene (formed using the peak molecular weight of standard polystyrene), based on the molecular weight of the peaks in the chromatogram measured by GPC, in the same manner as for the hydrogenated block copolymer (a) described above.

One polyphenylene ether resin may be used singly, or ones modified by blending with a resin such as a polystyrenic resin or polypropylene resin for improving the processability may be used.

Examples of the polystyrenic resin include, but are not limited to, general-purpose polystyrene (GPPS), impact-resistant polystyrene reinforced by rubber components (HIPS), styrene-butadiene copolymers, hydrogenated styrene-butadiene copolymers other than the hydrogenated block copolymer (P) used in the present embodiment, styrene-maleic anhydride copolymers, styrene-acrylonitrile copolymers, styrene-acrylonitrile-butadiene copolymers, and styrene-methyl methacrylate copolymers. These copolymers may be random copolymers or block copolymers.

Examples of the polypropylene resin include, but are not limited to, block copolymers or random copolymers of propylene with an olefin other than propylene, preferably an $\alpha$-olefin having 2 to 20 carbon atoms, or blends thereof.

As described above, when the polyphenylene ether resin is contained, the heat resistance, that is, the low compression set property can be improved. In the case where the elastomer composition is used as a medical plug, the resealability can be improved. When the hydrogenated block copolymer (P) of the present embodiment is used, the amount of the polyphenylene ether resin to be added may be reduced, or addition of the polyphenylene ether resin itself may be eliminated. Then, the amount of the non-aromatic softener to be added for adjustment of the hardness also can be reduced. It is also possible to reduce or eliminate the odor specific to the polyphenylene ether resin. In other words, the elastomer composition of the present embodiment may contain or may not contain the polyphenylene ether resin. In applications in which odors derived from the polyphenylene ether resin is not a problem or a sophisticated low compression set property is required, the polyphenylene ether resin may be contained. The content of the polyphenylene ether resin in this case is 5 parts by mass to 100 parts by mass, preferably 10 parts by mass to 90 parts by mass, more preferably 20 parts by mass to 85 parts by mass, and even more preferably 30 parts by mass to 85 parts by mass based on 100 parts by mass of the hydrogenated block copolymer (P).

When the content of the polyphenylene ether resin is within the range described above, the compression set property of the elastomer composition tends to be favorable.

The elastomer composition of the present embodiment may contain a hydrogenated styrenic elastomer other than the hydrogenated block copolymer (P) described above. The hydrogenated styrene elastomer is not limited to the following, and examples of typical hydrogenated styrenic elastomers include styrene - butadiene - styrene (SBS), styrene - ethylene - butylene - styrene (SEBS) obtained by saturating styrene - isoprene - styrene by hydrogenation, and styrene - ethylene - propylene - styrene (SEPS).

Additional examples include elastomers of such a structure as styrene - ethylene - butylene (SEB) or styrene - ethylene - propylene (SEP).

Moreover, reactive elastomers may be used, which are obtained by adding a variety of functional groups to the above hydrogenated styrenic elastomers.

Examples of the functional group include, but are not limited to, a hydroxyl group, a carboxyl group, a carbonyl group, a thiocarbonyl group, an acid halide group, an acid anhydride group, a thiocarboxylic acid group, an aldehyde group, a thioaldehyde group, a carboxylic acid ester group, an amide group, a sulfonic acid group, a sulfonic acid ester group, a phosphoric acid group, a phosphoric acid ester group, an amino group, an imino group, a nitrile group, a pyridyl group, a quinoline group, an epoxy group, a thioepoxy group, a sulfide group, an isocyanate group, an isothiocyanate group, a silicon halide group, an alkoxy silicon group, a tin halide group, a boronic acid group, a boron-containing group, a boronic acid salt group, an alkoxy tin group, and a phenyl tin group.

The elastomer composition of the present embodiment may be partially crosslinked in the presence of an organic peroxide from the viewpoint of the compression set property.

Examples of the organic peroxide include, but are not limited to, 2,5-dimethyl-2,5-di(t-butylperoxy)hexane, dicumyl peroxide, 2,5-dimethyl-2,5-di(benzoylperoxy)hexane, t-butylperoxy benzoate, t-butyl cumyl peroxide, diisopropylbenzene hydroxy peroxide, 1,3-bis(t-butylperoxyisopropyl)benzene, benzoyl peroxide, 1,1-di(t-butylperoxy)-3,3,5-trimethylcyclohexane, t-butyl hydroperoxide, 1,1,3,3-tetramethylbutyl hydroperoxide, cumene hydroperoxide, di-t-butyl peroxide, 1,1-di-t-butylperoxy-cyclohexane, 2,5-dimethyl-2,5-di(t-butylperoxy)hexyne-3, n-butyl-4,4-bis(t-butylperoxy)valerate, t-butylperoxy isobutyrate, t-butylperoxy-2-ethyl hexanoate, and t-butylperoxyisopropyl carbonate.

Only one of these may be used, or two or more of organic peroxides may be used in combination.

The amount of the organic peroxide to be used is preferably 0.05 parts by mass to 5 parts by mass, more preferably 0.1 parts by mass to 4 parts by mass, and even more preferably 0.3 parts by mass to 3 parts by mass based on 100 parts by mass of the hydrogenated block copolymer (P).

When the amount of the organic peroxide to be used is within the range, an elastomer composition excellent

in recovery properties, particularly a compression set property, tends to be obtained without decrease in the excellent processability.

When the elastomer composition of the present embodiment is partially crosslinked, a crosslinking aid may be used as required in order to adjust the degree of crosslinking.

Examples of the crosslinking aid include, but are not limited to, trimethylolpropane triacrylate, triallyl isocyanurate, triallyl cyanurate, triallyl formal, triallyl trimellitate, N,N'-m-phenylene-bis-maleimide, dipropargyl terephthalate, diallylphthalate, tetraallyl terephthalamide, triallyl phosphate, divinylbenzene, ethylene dimethacrylate, diallyl phthalate, quinonedioxime, ethylene glycol dimethacrylate, polyfunctional methacrylate monomers, polyhydric alcohol methacrylates and acrylates, and unsaturated silane compounds such as vinyltrimethoxysilane and vinyltriethoxysilane.

Only one of these may be used, or two or more of these may be used in combination as required.

The amount of the crosslinking aid to be used is preferably 0.1 parts by mass to 10 parts by mass, more preferably 0.2 parts by mass to 8 parts by mass, and even more preferably 0.5 parts by mass to 7 parts by mass based on 100 parts by mass of the hydrogenated block copolymer (P).

The elastomer composition of the present embodiment may contain additives other than components described above as long as the objective of the present embodiment is not impaired.

Examples of such additives include thermal stabilizers, antioxidants, ultraviolet radiation absorbers, anti-aging agents, plasticizers, photostabilizers, crystal nucleating agents, impact modifiers, pigments, lubricants, antistatic agents, flame retardants, flame-retardant aids, compatibilizers, and tackifiers.

Particularly, addition of silicone oil as a lubricant improves slidability and is effective for reduction in needlestick resistance and improvement in coring.

Examples of the type of silicone oil include common dimethylpolysiloxane and phenyl-methylpolysiloxane. Particularly, dimethylpolysiloxane is preferred.

The amount of silicone oil to be added is preferably 0.5 parts by mass to 10 parts by mass, more preferably 0.7 parts by mass to 7 parts by mass, and even more preferably 1.0 parts by mass to 5 parts by mass based on 100 parts by mass of the hydrogenated block copolymer (a). The kinematic viscosity of the silicone oil is not particularly limited and is preferably 10 $mm^2$/sec to 10000 $mm^2$/sec, more preferably 50 $mm^2$/sec to 7000 $mm^2$/sec, and even more preferably 100 $mm^2$/sec to 5000 $mm^2$/sec.

Only one of these additives may be used, or two or more of these may be used in combination.

(Method for producing elastomer composition)

The method for producing the elastomer composition of the present embodiment is not particularly limited, and conventionally known methods can be used.

Examples of the method include melt kneading methods involving a commonly used mixer such as a pressurizing kneader, a Banbury mixer, an internal mixer, a Laboplast mill, a Mix-Labo, a single screw extruder, a twin screw extruder, a co-kneader, or a multiscrew extruder, and methods in which components are dissolved or dispersion-mixed, and the solvent is then removed by heating.

When the elastomer composition of the present embodiment is partially crosslinked by the organic peroxide described above, compounding of each of the components and partial crosslinking of the organic peroxide (and a crosslinking aid to be added as required) may be conducted simultaneously, or after compounding of each of the components, partial crosslinking may be conducted with the organic peroxide, and a crosslinking aid, as required, added.

Alternatively, a portion of each of the components, the organic peroxide, and a crosslinking aid, as required, may be mixed and crosslinked, and then, the remaining components may be mixed thereto.

The partial crosslinking can be conducted under temperature conditions at which the organic peroxide being used undergoes decomposition, generally 150°C to 250°C.

In the case where compounding of some or all of each of the components and crosslinking by means of the organic peroxide (and a crosslinking aid to be added as required) are conducted simultaneously, the compounding can be conducted using the melt-kneader at a temperature at which the organic peroxide being used undergoes decomposition.

(Applications)

A seal member of the present embodiment comprises the elastomer composition described above. A plug body of the present embodiment comprises the elastomer composition described above. A medical plug for use in medical applications of the present embodiment comprises the elastomer composition described above.

(Plug body applications)

The elastomer composition of the present embodiment, when used in plug body applications, is preferably used in a medical airtight container or sealed container. In an application for the purpose of sticking an injection needle, a typical form is a columnar plug body that is fitted into a substantially cylindrical lid body or a predetermined jig and used integrally with the lid body or the jig.
Examples of the jig include, but are not limited to, predetermined frames, caps, housings, and encapsulants. Specific examples thereof include a form to be employed as a cap for transfusion bags.
The columnar plug body may also be used singly in some cases, where the plug body is fitted into the mouth of a glass bottle and functions as a sealable plug.
In applications for the purpose of not sticking an injection needle, a disk-shaped plug body is fitted into the inner surface of a lid for a container that can be sealed with screws or the like, and thereby contributes to improvement in sealability.
The plug body containing the elastomer composition of the present embodiment is preferably used as a plug, particularly as a plug for medical containers.
An airtight container or a sealed container is preferred as a medical container. Examples thereof include, but are not limited to, transfusion bags, peritoneal dialysis bags, transfusion bottles, transfusion soft bottles, glass vials, and plastic vials.
Examples of the shape of the plug body include, but are not particularly limited to, truncated cone, columnar, and disk shapes. The diameter thereof is usually on the order of from 5 mm to 25 mm. The thickness of the plug body, that is, the thickness thereof in the direction of insertion of an injection needle in applications for the purpose of sticking the injection needle, is not particularly limited and is usually on the order of from 2 mm to 10 mm.

(Method for producing plug body)

The plug body can be produced by, for example, but not particularly limited to, injection molding, compression, or punching from extrusion.
The plug body is preferably produced by injection molding from the viewpoint of dimensional accuracy and surface roughness reproducibility.

Examples

Below, the present embodiment will now be described in detail by way of specific Examples and Comparative Examples, but the present embodiment is not limited to Examples below. For example, in the present embodiment, a hydrogenated block polymer is used to prepare a composition, which is further made into a plug body of a specific shape. The plug body may be once melted, and the melted plug body may be remolded into a shape listed in Examples and subjected to similar evaluations. In this case, a method for quantifying the composition of the composition is not particularly limited and may be conducted by using common quantification techniques, such as GPC and NMR, in combination.
First, the evaluation methods and the methods for measuring physical properties applied to Examples and Comparative Examples will now be described below.

[Physical Properties of Hydrogenated Block Copolymer]

(Peak top molecular weight of diblock component in hydrogenated block copolymer and weight average molecular weight of total of hydrogenated block copolymer)

Each molecular weight was measured by gel permeation chromatography (GPC) [apparatus: manufactured by Waters Corporation] under the following conditions. The molecular weight of the peak top corresponding to the diblock component in the hydrogenated block copolymer was determined from the resultant chromatogram using a calibration curve obtained by measurement on commercially available standard polystyrene (formed using the peak molecular weight of standard polystyrene). A base line including all the peaks was set, and the weight average molecular weight of the total hydrogenated block copolymer was calculated in the same manner.

(Measurement conditions)

GPC; ACQUITY APC system (manufactured by Nihon Waters K.K.)

System (measurement/analysis) software; Empower 3
Detector; differential refractive index (RI) detector
Refractive index unit full scale; 500 μRIU
Output full scale; 2000 mV
Sampling rate; 10 points/sec
Column; ACQUITY APC XT125 (4.6 mm × 150 mm); 1
ACQUITY APC XT200 (4.6 mm × 150 mm); 1
ACQUITY APC XT900 (4.6 mm × 150 mm); 1
ACQUITY APC XT450 (4.6 mm × 150 mm); 1
Solvent; tetrahydrofuran (THF)
Flow rate; 1.0 mL/ min
Concentration; 0.1 mg/mL
Column temperature; 40°C
Injection volume; 20 μL

(Diblock component proportion, dibranched component proportion, tribranched component proportion, and tetra- or higher-branched component proportion in hydrogenated block copolymer)

The inflection point on each curve between the peaks obtained in the above GPC was vertically partitioned. The ratio of each of the partitioned areas to the total area was taken as the mass ratio of the corresponding component. When no inflection point was observed between the peaks, the ratio of the partitioned area from the position of a molecular weight 1.5 times the peak top molecular weight of the diblock component to the position of a molecular weight 2.5 times the peak top molecular weight of the diblock component relative to the total area of the peaks was taken as the dibranched component proportion. Similarly, the ratio of the partitioned area from the position of a molecular weight 2.5 times the peak top molecular weight of the diblock component to a position of a molecular weight 3.5 times the peak top molecular weight of the diblock component relative to the total area of the peaks was taken as the tribranched component proportion, and the ratio of the partitioned area of the component having a molecular weight 3.5 times or more the peak top molecular weight of the diblock component was taken as the tetra- or higher-branched component proportion.

(Content of total vinyl aromatic monomer units in hydrogenated block copolymer (total styrene content))

A given amount of the hydrogenated block copolymer (P) was dissolved in chloroform, and the solution was analyzed using an ultraviolet spectrophotometer (manufactured by SHIMADZU CORPORATION, UV-2450). The content of vinyl aromatic monomer units (styrene) was calculated using a calibration curve from the peak intensity of an absorption wavelength (262 nm) attributed to the vinyl aromatic compound component (styrene).

(Proportion of 1,2-bonds and 3,4-bonds in conjugate diene monomer unit in hydrogenated block copolymer)

The proportion of the 1,2-bonds and 3,4-bonds in the conjugate diene monomer unit in the hydrogenated block copolymer (P) was measured using a nuclear magnetic resonator (NMR) under the following conditions.
After the completion of all the reactions (for the hydrogenated block copolymer, after the completion of hydrogenation reaction), methanol was added in a large amount to the reaction solution to precipitate and recover the hydrogenated block copolymer (P). Subsequently, the hydrogenated block copolymer (P) recovered was extracted with acetone, and the extract was vacuum-dried and used as a sample for analysis. The conditions for measurement are as follows.

(Measurement Conditions)

Measurement instrument: JNM-LA400 (manufactured by JEOL Ltd.)
Solvent: Deuterated chloroform
Sample concentration: 50 mg/ml
Observation frequency: 400 MHz
Chemical shift reference: TMS (tetramethylsilane)
Pulse delay: 2.904 seconds
Number of scans: 64
Pulse width: 45°
Measurement temperature: 26°C

The proportion of the 1,2-bonds and 3,4-bonds in the conjugate diene monomer unit in the hydrogenated block copolymer was determined from the ratio of the total peak area of the 1,2-bonds and 3,4-bonds to the total area of all the peaks related to the conjugated diene monomer unit (1,2-bonds, 3,4-bonds, and 1,4-bonds) in the obtained peaks.

(Content of total vinyl aromatic monomer unit in hydrogenated block copolymer)

The content of the total vinyl aromatic monomer units in the hydrogenated block copolymer (P) was measured using a nuclear magnetic resonator (NMR) under the conditions as those for the method of measuring the (proportion of 1,2-bonds and 3,4-bonds).

(Degree of Hydrogenation in Conjugate Diene Monomer Unit in Hydrogenated Block Copolymer)

The degree of hydrogenation of double bonds in the conjugate diene monomer unit in the hydrogenated block copolymer (P) was measured using a nuclear magnetic resonator (NMR) under the conditions as those for the method of measuring the (proportion of 1,2-bonds and 3,4-bonds) .

The degree of hydrogenation of double bonds in the conjugate diene monomer unit in the hydrogenated block copolymer (P) was determined by calculating the ratio of the total peak area of the hydrogenated 1,2-bonds and hydrogenated 3,4-bonds and hydrogenated 1,4-bonds to the total area of all the peaks related to double bonds in the conjugated diene monomer unit (1,2-bonds, 3,4-bonds, and 1,4-bonds) in the obtained peaks.

[Evaluations of Hydrogenated Block Copolymer]

(Compression set at 70°C of hydrogenated block copolymer (p))

The compression set at 70°C of the hydrogenated block copolymer (P) was determined as follows, in accordance with JIS K6301 compression set test. A press sheet of 2 mm in thickness of the hydrogenated block copolymer (P) was punched into round pieces of 29 mm in diameter. Six round pieces were stacked to give a specimen. The initial thickness of the specimen of the 6 stacked pieces was measured at 23°C. Thereafter, the specimen was left in an oven of 70°C for 22 hours in a 25% compressed state. Then, the specimen was taken out of the oven. After the release from the compression, the specimen was left at 23°C for 30 minutes, and a residual strain rate (compression set at 70°C) was then determined by the following expression.

$$\text{Compression set } 70°\text{C} = (t_0 - t_1)/(t_0 \times 0.25) \times 100$$

$t_0$: The initial thickness of the specimen (mm)
$t_1$: The thickness of the specimen after released from the compression and left at 23°C for 30 minutes (mm)

The lower the residual strain rate, the more excellent the heat resistance and the mechanical physical properties. When the residual strain rate was 25% or less, the specimen was evaluated to be practically excellent.

(Shear melt viscosity of hydrogenated block copolymer (p))

The shear melt viscosity (hereinafter, also referred to as "capillary viscosity") of the hydrogenated block copolymer (P) was determined as follows, in accordance with ISO 11443. The measurement was conducted under conditions of a temperature of 240°C and a shear rate of 122 $sec^{-1}$. This capillary viscosity is a value obtained by measurement in accordance with ISO 11443, but is a so-called "apparent viscosity", which has not been subjected to the Bagley correction or the Rabinovitch correction. Since the measurement value greatly depends on measurement apparatus conditions, the measurement apparatus conditions were specified as follows.

Capillary die inner diameter: 1.0 mm
Capillary die length: 10.0 mm
Inlet angle: 180°
Piston diameter: 9.510 mm
Furnace body diameter: 9.55 mm

The lower the shear melt viscosity, the more excellent the processability. When the shear melt viscosity was 4000 mP·s or less under the conditions, the hydrogenated block copolymer (P) was evaluated to be practically excellent.

[Evaluations of Elastomer Composition]

[Production of Thermoplastic Elastomer Composition]

Raw material components were homogeneously mixed, and the mixture was melt-kneaded with a twin screw extruder ("TEX-30αII" manufactured by The Japan Steel Works, Ltd., cylinder bore diameter: 30 mm) at a temperature set at 230°C to thereby produce pellets of a thermoplastic elastomer composition.

[Production of Sheet]

Raw material components were homogeneously mixed, and the mixture was melt-kneaded with a twin screw extruder ("TEX-30aII" manufactured by The Japan Steel Works, Ltd., cylinder bore diameter: 30 mm) at a temperature set at 230°C. A thermoplastic elastomer composition sheet having a thickness of 500 μm was produced using a T die at a number of revolutions of the screws of 300 rpm.

(Amount of grains)

The thermoplastic elastomer composition sheet was cut into a 10 cm × 10 cm square and both the surfaces were observed with a microscope. The number of grains was counted and evaluated based on the following criteria.

The amount of the hydrogenated block copolymer in the composition sheet was not uniform. Thus, for the weight of composition sheet being X g and the amount of the hydrogenated block copolymer in the composition sheet being Y g, the total number of grains × Y/X, which was taken as the evaluation criteria, was rounded off to the nearest whole number.

Evaluation criteria of amount of grains

◎: The number of grains having a diameter of 50 μm or more is 0.
○: The number of grains having a diameter of 50 μm or more and less than 200 μm is 1 to 2, and the number of grains having a diameter of 200 μm or more is 0.
△: The number of grains having a diameter of 50 μm or more and less than 200 μm is 3 or more, and the number of grains having a diameter of 200 μm or more is 0.
∇: The number of grains having a diameter of 200 μm or more is 1 to 2.
×: The number of grains having a diameter of 200 μm or more is 3 or more.

The number of grains affects resealability and coring resistance. Thus, a composition sheet given ◎ was evaluated as extremely excellent, a composition sheet given O was evaluated as excellent, and a composition sheet given △ was evaluated as practically favorable.

[Production of Plug Body]

The pellets of the thermoplastic elastomer composition of obtained in [Production of Thermoplastic Elastomer Composition] described above was were molded into a columnar plug body 1 of 20 mm in diameter × 4 mm in thickness, using an injection molding machine FE120S18A (manufactured by Nissei Plastic Industrial Co., Ltd.).

FIG. 1(A) shows a schematic top view of the plug body 1, and FIG. 1(B) shows a schematic cross-sectional view of the plug body 1.

The injection molding conditions were as follows: resin temperature: 240°C, injection rate: 45 $cm^3$/sec, injection time: 10 seconds, mold temperature: 40°C, and cooling time: 40 seconds.

(Resealability after steam sterilization treatment)

First, the plug body 1 shown in FIG. 1 was fitted into a predetermined jig 2 as shown in FIG. 2.

Next, the resultant was fastened with another jig such that the lock ring 23 was in complete contact with the holder 22 with the plug body 1 fitted thereinto. Steam sterilization treatment was performed on the resultant at 121°C for 20 minutes at a steam pressure of 0.104 MPa in an autoclave model SN500 manufactured by Yamato Scientific Co., Ltd.

Then, the resultant was cooled for 2 hours in the apparatus, and the plug body 1 was taken out thereof and further cooled at room temperature (23°C) over 2 hours. Thereafter, in the same manner as in (Needlestick resistance)

described below, the plug body 1 of FIG. 1, fitted into the jig 2 of FIG. 2, was attached to the mouth plug part of a PET bottle filled with 500 mL of water, and the jig 2 was fastened.

The bottle was inverted such that the plug body portion was positioned on the lower side. In this case, the height from the inner surface of the plug body to the surface of the fluid was 18.5 mm.

A hole of 3 mm in diameter was opened at the bottom (upper side) of the bottle. A resin needle (plastic bottle needle) of 5 mm in diameter (maximum diameter at the base) was stuck, to a part with the maximum diameter of the needle, into the central part of the plug body 1.

The resultant was left as it was for 4 hours. The needle was removed from the plug body 1, and the mass of water leaked from the needlestick mark was measured.

When the mass of water leaked was smaller, it was determined that the plug body had more favorable resealability.

Eight measurements were simply averaged to obtain the measurement value. Resealability was evaluated with respect to the measurement value based on the following criteria.

◎: The mass of water leaked is 0 g.
○: The mass of water leaked is more than 0 g and 0.1 g or less.
△: The mass of water leaked is more than 0.1 g and 1.0 g or less.
▽: The mass of water leaked is more than 1.0 g and 5.0 g or less.
×: The mass of water leaked is more than 5.0 g.
A plug body given ◎ was evaluated as extremely excellent, a plug body given ○ was evaluated as excellent, and a plug body given △ was evaluated as practically favorable.

(Needlestick Resistance)

The plug body 1 shown in FIG. 1 was fitted into a predetermined jig as shown in FIG. 2.

FIG. 2(A) shows a schematic top view of the jig 2, and FIG. 2(B) shows a schematic cross-sectional view of the jig 2.

The jig 2 had a tubular holder 22 attachable via a screw pitch part 21 to the mouth of a predetermined container.

The plug body 1 was fitted into the end of the opening of the jig 2 and fastened using a lock ring 23.

Next, the jig 2 with the plug body 1 fitted thereinto was attached to the mouth plug part of a PET bottle filled with 500 mL of water, and fastened.

The PET bottle was mounted to a tensile tester TG-5 kN (manufactured by Minebea Co., Ltd.) such that the plug body 1 was in an upward direction. A resin needle (plastic bottle needle) of 5 mm in diameter (maximum diameter at the base) was allowed to penetrate through the central part of the plug body 1 from the upper side thereof at a speed of 200 mm/min. The maximum load in this operation was measured.

The needle used was Terufusion Infusion Set manufactured by Terumo Corporation (TK-U200L).

When the maximum load was smaller, it was determined that the plug body was more favorable with lower needlestick resistance.

Four measurements were simply averaged to obtain the measurement value. Needlestick resistance was evaluated with respect to the measurement value based on the following criteria.

◎: The maximum load is 3.0 kgf or less.
○: The maximum load is more than 3.0 kgf and 3.5 kgf or less.
△: The maximum load is more than 3.5 kgf and 4.0 kgf or less.
×: The maximum load was more than 4.0 kgf.
A plug body given ◎ was evaluated as extremely excellent, a plug body given ○ was evaluated as excellent, and a plug body given △ was evaluated as practically favorable.

(Coring resistance)

In the same manner as in (Needlestick resistance) described above, the plug body 1 of FIG. 1 was fitted into the jig 2 of FIG. 2, and then attached and fastened to the mouth plug part of a PET bottle filled with 500 mL of water.

A resin needle (plastic bottle needle) of 5 mm in diameter (maximum diameter at the base) was stuck into and removed from the central part of the plug body 1 in the bottle five times. Then, the presence or absence of shaves of the plug body in the content water or on the needle surface was visually observed.

When a plug body was free from shaves or had few shaves, it was determined that the plug body had favorable coring resistance.

Five measurements were simply averaged to obtain the measurement value. Coring resistance was evaluated

with respect to the measurement result based on the following criteria.

◎: No shave of the plug body is observed.
○: One to three shaves of the plug body in a size of 0.5 mm or less are observed, and no shave in a size of more than 0.5 mm is observed.
△: Four to five shaves of the plug body in a size of 0.5 mm or less are observed, and no shave in a size of more than 0.5 mm is observed.
∇: Six or more shaves of the plug body in a size of 0.5 mm or less are observed, and no shave in a size of more than 0.5 mm is observed.
×: One or more shaves of the plug body in a size of more than 0.5 mm are observed.
A plug body given ◎ was evaluated as particularly excellent, a plug body given O was evaluated as excellent, and a plug body given △ was evaluated as practically favorable.

(Odor sensory test)

The plug body 1 shown in FIG. 1 was placed in a 500 mL pressure-tight glass bottle, which was then sealed, heated at 70°C for 1 hour, and then left at room temperature for 48 hours.
Thereafter, 10 panelists examined odor from the mouth of the glass bottle and evaluated the odor based on the following <Odor Intensity>.
The average value of the following odor intensity was calculated and assessed based on the following criteria.

(Criteria)

A plug body having odor intensity of less than 3 was evaluated as practically favorable.

<Odor Intensity>

0: no odor, 1: slightly perceivable odor, 2: weak, but perceivable what the odor is from, 2.5: intermediate between 2 and 3, 3: easily perceivable odor, 3.5: intermediate between 3 and 4, 4: strong odor, and 5: intense odor

(Oil bleed resistance)

The plug body 1 shown in FIG. 1 was placed on kraft paper, and a load of 250 g was applied from upward. The plug body 1 was left to stand in a gear oven at 180°C for 72 hours. Thereafter, a colorimetric color difference meter ZE6000 (manufactured by Nippon Denshoku Industries Co., Ltd.) was used to measure the brightness L* before and after the test. The difference ΔL* (brightness before test L* - brightness after test L*) was determined and evaluated based on the following criteria.

◎: -1.0 or more
○: -1.5 or more and less than -1.0
△: -2.0 or more and less than -1.5
×: less than -2.0
A plug body given ◎ was evaluated as particularly excellent, a plug body given O was evaluated as excellent, and a plug body given △ was evaluated as practically favorable.

(Production of hydrogenated block copolymer)

[Example 1]

<Preparation of Hydrogenation Catalyst>

A hydrogenation catalyst used for preparing a hydrogenated block copolymer composition in Examples and Comparative Examples described below was prepared according to the following method.
A reaction container equipped with a stirring apparatus was purged with nitrogen in advance and charged with 1 L of dried and purified cyclohexane.
Next, 100 mmol of bis(η5-cyclopentadienyl)titanium dichloride was added thereto.
While the contents were thoroughly stirred, a n-hexane solution containing 200 mmol of trimethylaluminum was added thereto. The mixture was reacted at room temperature for approximately 3 days to obtain a hydrogenation catalyst.

<Production of Hydrogenated Block Copolymer>

A vessel-type reactor having an internal capacity: 100 L equipped with a stirring apparatus and a jacket was washed, dried, and purged with nitrogen, and batch polymerization was performed as follows to produce a hydrogenated block copolymer.

As a first step, the reactor was charged with a cyclohexane solution containing 38 L of cyclohexane and 20.0 parts by mass of a styrene monomer, and then, 1.2 mol of N,N,N',N'-tetramethylethylenediamine (hereinafter, also referred to as "TMEDA") was added thereto per 1 mol of n-butyl lithium (hereinafter, also referred to as "Bu-Li") .

As a second step, after the temperature was adjusted to 40°C, 0.056 parts by mass of Bu-Li were added based on 100 parts by mass of the total monomers, and polymerization was performed at a temperature inside the reactor of 45°C for 30 minutes.

As a third step, a cyclohexane solution containing 80.0 parts by mass of a conjugated diene monomer (butadiene monomer) was introduced thereto, and thereafter, polymerization was performed for 60 minutes while the reaction temperature was adjusted to 80°C.

As a fourth step, tetramethoxysilane (hereinafter, also referred to as "TMS") was added such that the molar ratio of Si to Li (Si/Li) was 0.24 mol. After stirring for 20 minutes, 0.1 mol of methanol was added per 1 mol of Bu-Li to obtain a styrene-butadiene coupled polymer.

As a fifth step, the hydrogenation catalyst prepared as described above was used to continuously hydrogenate the coupled polymer obtained at 95°C to thereby obtain a hydrogenated block copolymer 1. The amount of the catalyst was 100 ppm, the hydrogen pressure in the hydrogenation polymerization reactor was 0.95 MPa, and the average residence time was 120 minutes. After the reaction was completed, 0.25 parts by mass of an antioxidant (octadecyl-3-(3,5-dibutyl-t-butyl-4-hydroxyphenyl)propionate) were added thereto per 100 parts by mass of the hydrogenated block copolymer 1 to obtain a polymer 1. The physical properties and characteristics of the obtained polymer 1 were each measured by the methods described above. The measurement results are shown in Table 1.

[Examples 2, 3, and 8 and Comparative Examples 4, 5, 10, and 11]

Polymers 2, 3, 8, 14, 15, 20, and 21 were obtained in the same manner as in Example 1 except that the amount of TMS added was adjusted as shown in Tables 1 and 2 in the fourth step. The physical properties and characteristics of the obtained polymer were each measured by the methods described above. The measurement results are shown in Tables 1 and 2.

[Examples 4 and 5 and Comparative Examples 6 and 7]

Polymers 4, 5, 16, and 17 were obtained in the same manner as in Example 1 except that the amount of Bu-Li added was adjusted as shown in Tables 1 and 2 in the second step. The physical properties and characteristics of the obtained polymer were each measured by the methods described above. The measurement results are shown in Tables 1 and 2.

[Examples 6 and 7 and Comparative Examples 8 and 9]

Polymers 6, 7, 18, and 19 were obtained in the same manner as in Example 1 except that the amount of the styrene monomer was adjusted as shown in Tables 1 and 2 in the first step and the amount of the conjugated diene monomer (butadiene monomer) was adjusted as shown in Tables 1 and 2 in the third step. The physical properties and characteristics of the obtained polymer were each measured by the methods described above. The measurement results are shown in Tables 1 and 2.

[Examples 9 and 10 and Comparative Example 12]

Polymers 9, 10, and 22 were obtained in the same manner as in Example 1 except that the amount of TMEDA was adjusted as shown in Tables 1 and 2 in the first step. The physical properties and characteristics of the obtained polymer were each measured by the methods described above. The measurement results are shown in Tables 1 and 2.

[Comparative Example 1]

A polymer 11 was obtained in the same manner as in Example 1 except that the amount of TMEDA added was adjusted as shown in Table 2 in the first step, the amount of Bu-Li added was adjusted as shown in Table 2 in the second step, and the amount of TMS added was changed to 0.45 as shown in Table 2 in the fourth step. The physical properties

and characteristics of the obtained polymer were each measured by the methods described above. The measurement results are shown in Table 2.

[Comparative Examples 2 and 3]

Polymers 12 and 13 were obtained in the same manner as in Example 1 except that the amount of TMEDA added and the amount of the styrene monomer were adjusted as shown in Table 2 in the first step, the amount of Bu-Li added was adjusted as shown in Table 2 in the second step, the amount of the conjugated diene monomer (butadiene monomer) was adjusted as shown in Table 2 in the third step, and TMS was replaced by a styrene monomer in the fourth step. The physical properties and characteristics of the obtained polymer were each measured by the methods described above. The measurement results are shown in Table 2.

[Comparative Example 13]

Polymer 23 was obtained in the same manner as in Example 1 except that the average residence time was changed to 90 minutes in the fifth step. The physical properties and characteristics of the obtained polymer were each measured by the methods described above. The measurement results are shown in Table 2.

[Comparative Example 14]

A polymer 24 was obtained in the same manner as in Example 1 except that the amount of the styrene monomer was adjusted as shown in Table 2 in the first step, the amount of Bu-Li added was adjusted as shown in Table 2 in the second step, and 30 parts of an isoprene monomer as a conjugated diene monomer were added in addition to 30 parts of the butadiene monomer in the third step. The physical properties and characteristics of the obtained polymer were each measured by the methods described above. The measurement results are shown in Table 2.

[Table 1]

| | Example 1 | Example 2 | Example 3 | Example 4 | Example 5 | Example 6 | Example 7 | Example 8 | Example 9 | Example 10 |
|---|---|---|---|---|---|---|---|---|---|---|
| Amount of styrene monomer in 1st step (parts by mass) | 20 | 20 | 20 | 20 | 20 | 14 | 38 | 20 | 20 | 20 |
| Amount of TMEDA in 1st step (mol) | 1.2 | 1.2 | 1.2 | 1.2 | 1.2 | 1.2 | 1.2 | 1.2 | 1.0 | 1.6 |
| Amount of Bu-Li added in 2nd step (parts by mass) | 0.056 | 0.056 | 0.056 | 0.060 | 0.044 | 0.056 | 0.056 | 0.056 | 0.056 | 0.056 |
| Amount of butadiene monomer in 3rd step (parts by mass) | 80 | 80 | 80 | 80 | 80 | 86 | 62 | 80 | 80 | 80 |
| Amount of TMS added in 4th step (Si/Li) | 0.24 | 0.27 | 0.3 | 0.24 | 0.24 | 0.24 | 0.24 | 0.3 | 0.24 | 0.24 |
| Average residence time in 5th step (minutes) | 120 | 120 | 120 | 120 | 120 | 120 | 120 | 120 | 120 | 120 |

(continued)

| Type of polymer | Polymer 1 | Polymer 2 | Polymer 3 | Polymer 4 | Polymer 5 | Polymer 6 | Polymer 7 | Polymer 8 | Polymer 9 | Polymer 10 |
|---|---|---|---|---|---|---|---|---|---|---|
| Structure of hydrogenated block copolymer | (A-B)n-X | (A-B)n-X | (A-B)n-X | (A-B)n-X | (A-B)n-X | (A-B)n-X | (A-B)n-X | (A-B)n-X | (A-B)n-X | (A-B)n-X |
| Content of vinyl aromatic monomer unit (mass%) | 20 | 20 | 20 | 20 | 20 | 14 | 38 | 20 | 20 | 20 |
| Content of conjugate diene monomer unit (mass%) | 80 | 80 | 80 | 80 | 80 | 86 | 62 | 80 | 80 | 80 |
| Vinyl bond content of conjugate diene monomer unit (mol%) | 65 | 63 | 63 | 63 | 63 | 65 | 65 | 65 | 53 | 75 |
| Degree of hydrogenation in conjugate diene monomer unit (mol%) | 99 | 99 | 99 | 99 | 99 | 99 | 99 | 99 | 99 | 99 |
| Diblock component proportion (mass%) | 29 | 18 | 18 | 27 | 28 | 22 | 22 | 15 | 22 | 22 |
| Dibranched component proportion (mass%) | 14 | 15 | 23 | 15 | 15 | 19 | 18 | 21 | 16 | 20 |
| Tribranched component proportion (mass%) | 50 | 62 | 55 | 50 | 51 | 53 | 52 | 57 | 53 | 51 |
| Tetra- or higher-branched component proportion (mass%) | 7 | 5 | 4 | 8 | 6 | 6 | 8 | 7 | 9 | 7 |
| Total (mass%) | 100 | 100 | 100 | 100 | 100 | 100 | 100 | 100 | 100 | 100 |
| Tribranched proportion/ dibranched proportion = M | 3.6 | 4.1 | 2.4 | 3.3 | 3.4 | 2.8 | 2.9 | 2.7 | 3.3 | 2.6 |

(continued)

| Type of polymer | Polymer 1 | Polymer 2 | Polymer 3 | Polymer 4 | Polymer 5 | Polymer 6 | Polymer 7 | Polymer 8 | Polymer 9 | Polymer 10 |
|---|---|---|---|---|---|---|---|---|---|---|
| Peak top molecular weight of diblock component (10 thousand) | 18.0 | 18.0 | 18.4 | 16.3 | 21.8 | 18.2 | 18.5 | 17.7 | 17.9 | 18.0 |
| Weight average molecular weight of total hydrogenated block copolymer (10 thousand) | 40.7 | 43.4 | 42.8 | 37.0 | 48.7 | 42.0 | 43.2 | 43.0 | 42.3 | 41.6 |
| Shear melt viscosity (mP·s) | 3290 | 3410 | 3310 | 2980 | 4000 | 3010 | 3770 | 3850 | 3800 | 2840 |
| Compression set at 70°C (%) | 21 | 19 | 25 | 25 | 19 | 25 | 23 | 19 | 21 | 25 |

[Table 2]

| | Ex. 1 | Comp. Ex. 1 | Comp. Ex. 2 | Comp. Ex. 3 | Comp. Ex. 4 | Comp. Ex. 5 | Comp. Ex. 6 | Comp. Ex. 7 | Comp. Ex. 8 | Comp. Ex. 9 | Comp. Ex. 10 | Comp. Ex. 11 | Comp. Ex. 12 | Comp. Ex. 13 | Comp. Ex. 14 |
|---|---|---|---|---|---|---|---|---|---|---|---|---|---|---|---|
| Amount of styrene monomer in 1st step (parts by mass) | 20 | 30 | 30 | 30 | 20 | 20 | 20 | 20 | 10 | 43 | 20 | 20 | 20 | 20 | 40 |
| Amount of TMEDA in 1st step (mol) | 1.2 | 0.5 | 1.2 | 0.5 | 1.2 | 1.2 | 1.2 | 1.2 | 1.2 | 1.2 | 1.2 | 1.2 | 0.65 | 1.2 | 1.2 |
| Amount of Bu-Li added in 2nd step (parts by mass) | 0.056 | 0.037 | 0.04 | 0.022 | 0.056 | 0.056 | 0.057 | 0.041 | 0.056 | 0.056 | 0.056 | 0.056 | 0.056 | 0.056 | 0.078 |
| Amount of conjugated diene monomer in 3rd step (parts by mass) | 80 | 70 | 70 | 70 | 80 | 80 | 80 | 80 | 90 | 57 | 80 | 80 | 80 | 80 | 60 |
| Amount of TMS added in 4th step (Si/Li) | 0.24 | 0.45 | 0 | 0 | 0.22 | 0.3 | 0.24 | 0.24 | 0.24 | 0.24 | 0.35 | 0.27 | 0.24 | 0.24 | 0.24 |
| Average residence time in 5th step (minutes) | 120 | 120 | 120 | 120 | 120 | 120 | 120 | 120 | 120 | 120 | 120 | 120 | 120 | 90 | 120 |
| Type of polymer | Polymer 1 | Polymer 11 | Polymer 12 | Polymer 13 | Polymer 14 | Polymer 15 | Polymer 16 | Polymer 17 | Polymer 18 | Polymer 19 | Polymer 20 | Polymer 21 | Polymer 22 | Polymer 23 | Polymer 24 |
| Structure of hydrogenated block copolymer | (A-B)n-X | (A-B)n-X | A-B-A | A-B-A | (A-B)n-X | (A-B)n-X | (A-B)n-X | (A-B)n-X | (A-B)n-X | (A-B)n-X | (A-B)n-X | (A-B)n-X | (A-B)n-X | (A-B)n-X | (A-B')n-X |

(continued)

| Type of polymer | Polymer 1 | Polymer 11 | Polymer 12 | Polymer 13 | Polymer 14 | Polymer 15 | Polymer 16 | Polymer 17 | Polymer 18 | Polymer 19 | Polymer 20 | Polymer 21 | Polymer 22 | Polymer 23 | Polymer 24 |
|---|---|---|---|---|---|---|---|---|---|---|---|---|---|---|---|
| Content of vinyl aromatic monomer unit (mass%) | 20 | 30 | 31 | 29 | 20 | 20 | 20 | 20 | 8 | 43 | 20 | 20 | 20 | 20 | 41 |
| Content of conjugate diene monomer unit (mass%) | 80 | 70 | 69 | 71 | 80 | 80 | 80 | 80 | 92 | 57 | 80 | 80 | 80 | 80 | 59 |
| Vinyl bond content of conjugate diene monomer unit (mol%) | 65 | 38 | 63 | 34 | 63 | 63 | 63 | 63 | 65 | 65 | 65 | 65 | 46 | 65 | 10 |
| Degree of hydrogenation in conjugate diene monomer unit (mol%) | 99 | 99 | 99 | 99 | 99 | 99 | 99 | 99 | 99 | 99 | 99 | 99 | 99 | 78 | 99 |
| Diblock component proportion (mass%) | 29 | 9 | 0 | 0 | 38 | 20 | 27 | 27 | 24 | 26 | 6 | 19 | 24 | 26 | 23 |
| Dibranched component proportion (mass%) | 14 | 71 | 100 | 100 | 17 | 33 | 16 | 13 | 19 | 15 | 35 | 16 | 18 | 15 | 6 |

(continued)

| Type of polymer | Polymer 1 | Polymer 11 | Polymer 12 | Polymer 13 | Polymer 14 | Polymer 15 | Polymer 16 | Polymer 17 | Polymer 18 | Polymer 19 | Polymer 20 | Polymer 21 | Polymer 22 | Polymer 23 | Polymer 24 |
|---|---|---|---|---|---|---|---|---|---|---|---|---|---|---|---|
| Tribranched component proportion (mass%) | 50 | 17 | 0 | 0 | 36 | 43 | 50 | 53 | 52 | 51 | 50 | 49 | 51 | 51 | 70 |
| Tetra- or higher-branched component proportion (mass%) | 7 | 3 | 0 | 0 | 9 | 4 | 7 | 7 | 5 | 8 | 9 | 16 | 7 | 8 | 1 |
| Total (mass%) | 100 | 100 | 100 | 100 | 100 | 100 | 100 | 100 | 100 | 100 | 100 | 100 | 100 | 100 | 100 |
| Tribranched proportion/ dibranched proportion = M | 3.6 | 0.2 | 0.0 | 0.0 | 2.1 | 1.3 | 3.1 | 4.1 | 2.7 | 3.4 | 1.4 | 3.1 | 2.8 | 3.4 | 11.7 |
| Peak top molecular weight of diblock component (10 thousand) | 18.0 | 24.0 | - | - | 18.0 | 18.4 | 15.0 | 23.0 | 18.2 | 18.5 | 17.7 | 15.3 | 15.4 | 23.6 | 11.6 |
| Weight average molecular weight of total hydrogenated block copolymer (10 thousand) | 40.7 | 48.8 | 22.0 | 44.0 | 36.9 | 40.4 | 33.8 | 52.4 | 41.2 | 42.4 | 44.1 | 38.1 | 35.3 | 54.0 | 33.7 |
| Shear melt viscosity (mP·s) | 3290 | 5540 | 3420 | 5650 | 3220 | 3115 | 3010 | 4580 | 3105 | 4020 | 4380 | 3540 | 4780 | 3740 | 3400 |

(continued)

| Type of polymer | Polymer 1 | Polymer 11 | Polymer 12 | Polymer 13 | Polymer 14 | Polymer 15 | Polymer 16 | Polymer 17 | Polymer 18 | Polymer 19 | Polymer 20 | Polymer 21 | Polymer 22 | Polymer 23 | Polymer 24 |
|---|---|---|---|---|---|---|---|---|---|---|---|---|---|---|---|
| Compression set at 70°C (%) | 21 | 35 | 45 | 17 | 33 | 34 | 33 | 19 | 39 | 27 | 21 | 30 | 20 | 41 | 41 |

[Production of Thermoplastic Elastomer Composition]

The following components were used as the raw materials for the thermoplastic elastomer composition.

<Hydrogenated Block Copolymer (a)>

The polymers polymerized in Examples and Comparative Examples were used as the hydrogenated block copolymer (a) .

<Polypropylene Resin (b)>

The following commercially available product was used as the polypropylene resin (b).

Polypropylene resin (b): SunAllomer Ltd., PM801A, propylene homopolymer, MFR (230°C, 2.16 kg) 13 g/10 min

<Non-aromatic Softener (c)>

The following commercially available products were used as the non-aromatic softener (c).

Non-aromatic softener (c-1): Diana Process Oil PW380 manufactured by Idemitsu Kosan Co., Ltd., paraffin oil, weight average molecular weight: 750, kinematic viscosity (40°C) = 380 $mm^2$/sec
Non-aromatic softener (c-2): Diana Process Oil PW90 manufactured by Idemitsu Kosan Co., Ltd., paraffin oil, weight average molecular weight: 530, kinematic viscosity (40°C) = 90.5 $mm^2$/sec

<Inorganic Filler (d)>

The following commercially available product was used as the inorganic filler (d).
Inorganic filler (d): Aerosil R972V manufactured by Nippon Aerosil Co., Ltd., average primary particle size: 16 nm, BET specific surface area: 110 $m^2$/g, dimethylsilyl-surface-treated silica

<Silicone Oil (e)>

The following commercially available product was used as the silicone oil (e).
Silicone oil (e): SH200·100Cs manufactured by Dow Corning Toray Co., Ltd., dimethylpolysiloxane, kinematic viscosity: 100 $mm^2$/sec

<Polyphenylene Ether Resin (f)>

The polyphenylene ether resin (f) was produced according to the following method.

Polyphenylene ether was polymerized according to oxidative coupled polymerization of 2,6-dimethylphenol based on a known method, and the resultant was purified to obtain the polyphenylene ether resin (f).

The reduced viscosity ($\eta$sp/c) (0.5 g/dL chloroform solution, measurement at 30°C), number average molecular weight, and average particle size of the obtained polyphenylene ether resin (f) are shown below.
Polyphenylene ether resin (f): reduced viscosity = 0.45 dL/g, number average molecular weight = 17,400, average particle size = 290 $\mu$m

(Examples 11 to 42 and Comparative Examples 15 to 34)

The polymer obtained as the hydrogenated block copolymer (a) in each of Examples 1 and 3 and Comparative Examples 1, 2, 4, 5, and 14 and the components (b) to (f) described above were blended as shown in Tables 3 to 11 to produce thermoplastic elastomer composition pellets according to the method described above. The obtained thermoplastic elastomer composition pellets were used to produce sheets of 500 $\mu$m in thickness and columnar plug bodies 1 of 20 mm in diameter $\times$ 4 mm in thickness as shown in FIG. 1 according to the above method. The amount of grains of each sheet obtained and characteristics of each plug body obtained were evaluated as described above. The evaluation results were shown in Tables 3 to 11.

[Table 3]

| | | Example 11 | Example 12 | Example 13 | Example 14 | Example 15 | Example 16 |
|---|---|---|---|---|---|---|---|
| Hydrogenated block copolymer | (a) Type of polymer | Polymer 1 | Polymer 1 | Polymer 1 | Polymer 3 | Polymer 3 | Polymer 3 |
| | Amount blended (parts by mass) | 100 | 100 | 100 | 100 | 100 | 100 |
| Polypropylene resin | (b) (parts by mass) | 35 | 35 | 35 | 35 | 35 | 35 |
| Polyphenylene ether resin | (f) (parts by mass) | 0 | 20 | 0 | 0 | 20 | 0 |
| Non-aromatic softener | (c-1) (parts by mass) | 55 | 60 | 30 | 55 | 60 | 30 |
| | (c-2) (parts by mass) | 105 | 120 | 60 | 105 | 120 | 60 |
| Inorganic filler | (d-1) (parts by mass) | 30 | 0 | 0 | 25 | 0 | 0 |
| Silicone oil | (e) (parts by mass) | 3 | 0 | 0 | 3 | 0 | 0 |
| Sheet characteristics | Amount of grains | ◎ | ◎ | ◎ | ○ | ○ | ○ |
| Plug body characteristics | Resealability after steam sterilization treatment | ◎ | ◎ | ◎ | ○ | ○ | ○ |
| | Needlestick resistance | ○ | ○ | ○ | ○ | ○ | ○ |
| | Coring resistance | ◎ | ○ | ○ | ○ | ○ | ○ |
| | Odor sensory test | 0.0 | 1.5 | 0.0 | 0.0 | 1.5 | 0.0 |
| | Oil bleed resistance | Δ | Δ | ○ | ○ | Δ | ○ |

[Table 4]

| | | Comparative Examples 15 | Comparative Examples 16 | Comparative Examples 17 | Comparative Examples 18 | Comparative Examples 19 |
|---|---|---|---|---|---|---|
| Hydrogenated block copolymer | (a) Type of polymer | Polymer 11 | Polymer 11 | Polymer 11 | Polymer 12 | Polymer 12 |
| | Amount blended (parts by mass) | 100 | 100 | 100 | 100 | 100 |
| Polypropylene resin | (b) (parts by mass) | 35 | 35 | 35 | 35 | 35 |
| Polyphenylene ether resin | (f) (parts by mass) | 0 | 20 | 0 | 0 | 20 |
| Non-aromatic softener | (c-1) (parts by mass) | 55 | 60 | 30 | 55 | 60 |
| | (c-2) (parts by mass) | 105 | 120 | 60 | 105 | 120 |
| Inorganic filler | (d-1) (parts by mass) | 25 | 0 | 0 | 25 | 0 |

(continued)

| | | Comparative Examples 15 | Comparative Examples 16 | Comparative Examples 17 | Comparative Examples 18 | Comparative Examples 19 |
|---|---|---|---|---|---|---|
| Silicone oil | (e) (parts by mass) | 3 | 0 | 0 | 3 | 0 |
| Sheet characteristics | Amount of grains | × | × | × | ◎ | ◎ |
| Plug body characteristics | Resealability after steam sterilization treatment | Δ | Δ | Δ | ∇ | ∇ |
| | Needlestick resistance | Δ | Δ | Δ | ◎ | ◎ |
| | Coring resistance | ∇ | ∇ | ∇ | ∇ | ∇ |
| | Odor sensory test | 0.0 | 1.5 | 0.0 | 0.0 | 1.5 |
| | Oil bleed resistance | × | × | × | Δ | Δ |

[Table 5]

| | | Comparative Examples 20 | Comparative Examples 21 | Comparative Examples 22 | Comparative Examples 23 | Comparative Examples 24 |
|---|---|---|---|---|---|---|
| Hydrogenated block copolymer | (a) Type of polymer | Polymer 12 | Polymer 14 | Polymer 14 | Polymer 14 | Polymer 15 |
| | Amount blended (parts by mass) | 100 | 100 | 100 | 100 | 100 |
| Polypropylene resin | (b) (parts by mass) | 35 | 35 | 35 | 35 | 35 |
| Polyphenylene ether resin | (f) (parts by mass) | 0 | 0 | 20 | 0 | 0 |
| Non-aromatic softener | (c-1) (parts by mass) | 30 | 55 | 60 | 30 | 55 |
| | (c-2) (parts by mass) | 60 | 105 | 120 | 60 | 105 |
| Inorganic filler | (d-1) (parts by mass) | 0 | 25 | 0 | 0 | 25 |
| Silicone oil | (e) (parts by mass) | 0 | 3 | 0 | 0 | 3 |
| Sheet characteristics | Amount of grains | ◎ | ◎ | ◎ | ◎ | ◎ |

(continued)

| | | Comparative Examples 20 | Comparative Examples 21 | Comparative Examples 22 | Comparative Examples 23 | Comparative Examples 24 |
|---|---|---|---|---|---|---|
| Plug body characteristics | Resealability after steam sterilization treatment | ▽ | ○ | ○ | ○ | ○ |
| | Needlestick resistance | ○ | Δ | Δ | ○ | ○ |
| | Coring resistance | Δ | ▽ | ▽ | ▽ | ▽ |
| | Odor sensory test | 0.0 | 0.0 | 1.5 | 0.0 | 0.0 |
| | Oil bleed resistance | ○ | × | Δ | ○ | Δ |

[Table 6]

| | | Comparative Examples 25 | Comparative Examples 26 | Comparative Examples 27 | Comparative Examples 28 | Comparative Examples 29 |
|---|---|---|---|---|---|---|
| Hydrogenated block copolymer | (a) Type of polymer | Polymer 15 | Polymer 15 | Polymer 24 | Polymer 24 | Polymer 24 |
| | Amount blended (parts by mass) | 100 | 100 | 100 | 100 | 100 |
| Polypropylene resin | (b) (parts by mass) | 35 | 35 | 35 | 35 | 35 |
| Polyphenylene ether resin | (f) (parts by mass) | 20 | 0 | 0 | 20 | 0 |
| Non-aromatic softener | (c-1) (parts by mass) | 60 | 30 | 55 | 60 | 30 |
| | (c-2) (parts by mass) | 120 | 60 | 105 | 120 | 60 |
| Inorganic filler | (d-1) (parts by mass) | 0 | 0 | 25 | 0 | 0 |
| Silicone oil | (e) (parts by mass) | 0 | 0 | 3 | 0 | 0 |
| Sheet characteristics | Amount of grains | ◎ | ◎ | × | × | × |

(continued)

| | | Comparative Examples 25 | Comparative Examples 26 | Comparative Examples 27 | Comparative Examples 28 | Comparative Examples 29 |
|---|---|---|---|---|---|---|
| Plug body characteristics | Resealability after steam sterilization treatment | ○ | ○ | ○ | ○ | ◎ |
| | Needlestick resistance | ○ | ○ | ◎ | ◎ | ○ |
| | Coring resistance | ▽ | ▽ | ▽ | ▽ | ▽ |
| | Odor sensory test | 1.5 | 0.0 | 0.0 | 1.5 | 0.0 |
| | Oil bleed resistance | Δ | ○ | × | × | Δ |

[Table 7]

| | | Example 17 | Example 18 | Example 19 | Example 20 | Example 21 | Example 22 |
|---|---|---|---|---|---|---|---|
| Hydrogenated block copolymer | (a) Type of polymer | Polymer 1 | Polymer 1 | Polymer 1 | Polymer 1 | Polymer 1 | Polymer 1 |
| | Amount blended (parts by mass) | 100 | 100 | 100 | 100 | 100 | 100 |
| Polypropylene resin | (b) (parts by mass) | 40 | 50 | 50 | 15 | 30 | 25 |
| Polyphenylene ether resin | (f) (parts by mass) | 0 | 0 | 0 | 0 | 0 | 0 |
| Non-aromatic softener | (c-1) (parts by mass) | 20 | 10 | 5 | 60 | 35 | 40 |
| | (c-2) (parts by mass) | 40 | 20 | 13 | 120 | 75 | 90 |
| Inorganic filler | (d-1) (parts by mass) | 0 | 0 | 0 | 0 | 0 | 0 |
| Silicone oil | (e) (parts by mass) | 0 | 0 | 0 | 0 | 0 | 0 |
| Sheet characteristics | Amount of grains | ◎ | ○ | ○ | Δ | ○ | Δ |
| Plug body characteristics | Resealability after steam sterilization treatment | ◎ | ○ | ○ | Δ | ○ | Δ |
| | Needlestick resistance | ○ | Δ | Δ | Δ | ○ | ○ |
| | Coring resistance | ○ | ○ | Δ | Δ | ○ | Δ |
| | Odor sensory test | 0.0 | 0.0 | 0.0 | 1.0 | 0 | 0 |
| | Oil bleed resistance | ○ | ◎ | ◎ | Δ | ○ | Δ |

[Table 8]

| | | Example 23 | Example 24 | Example 25 | Example 26 | Example 27 | Example 28 | Example 29 |
|---|---|---|---|---|---|---|---|---|
| Hydrogenated block copolymer | (a) Type of polymer | Polymer 1 | Polymer 1 | Polymer 1 | Polymer 1 | Polymer 1 | Polymer 1 | Polymer 1 |
| | Amount blended (parts by mass) | 100 | 100 | 100 | 100 | 100 | 100 | 100 |
| Polypropylene resin | (b) (parts by mass) | 50 | 50 | 50 | 50 | 30 | 20 | 10 |
| Polyphenylene ether resin | (f) (parts by mass) | 0 | 0 | 0 | 0 | 0 | 0 | 0 |
| Non-aromatic softener | (c-1) (parts by mass) | 30 | 20 | 15 | 8 | 65 | 80 | 100 |
| | (c-2) (parts by mass) | 50 | 40 | 25 | 17 | 125 | 150 | 180 |
| Inorganic filler | (d-1) (parts by mass) | 30 | 30 | 30 | 30 | 30 | 30 | 30 |
| Silicone oil | (e) (parts by mass) | 3 | 3 | 3 | 3 | 3 | 3 | 3 |
| Sheet characteristics | Amount of grains | ◎ | ◎ | ○ | ○ | Δ | Δ | Δ |
| Plug body characteristics | Resealability after steam sterilization treatment | ◎ | ○ | Δ | Δ | ○ | Δ | Δ |
| | Needlestick resistance | ○ | ○ | Δ | Δ | ○ | Δ | Δ |
| | Coring resistance | ◎ | ○ | ○ | Δ | ○ | Δ | Δ |
| | Odor sensory test | 0 | 0 | 0 | 0 | 0 | 0 | 0 |
| | Oil bleed resistance | ○ | ○ | ○ | ◎ | Δ | Δ | Δ |

[Table 9]

| | | Example 30 | Example 31 | Example 32 | Example 33 | Example 34 | Example 35 | Example 36 |
|---|---|---|---|---|---|---|---|---|
| Hydrogenated block copolymer | (a) Type of polymer | Polymer 1 | Polymer 1 | Polymer 1 | Polymer 1 | Polymer 1 | Polymer 1 | Polymer 1 |
| | Amount blended (parts by mass) | 100 | 100 | 100 | 100 | 100 | 100 | 100 |
| Polypropylene resin | (b) (parts by mass) | 50 | 50 | 50 | 50 | 30 | 20 | 10 |

(continued)

| | | Example 30 | Example 31 | Example 32 | Example 33 | Example 34 | Example 35 | Example 36 |
|---|---|---|---|---|---|---|---|---|
| Polyphenylene ether resin | (f) (parts by mass) | 20 | 20 | 20 | 20 | 20 | 20 | 20 |
| Non-aromatic softener | (c-1) (parts by mass) | 30 | 20 | 15 | 8 | 65 | 80 | 100 |
| | (c-2) (parts by mass) | 50 | 40 | 25 | 17 | 125 | 150 | 180 |
| Inorganic filler | (d-1) (parts by mass) | 0 | 0 | 0 | 0 | 0 | 0 | 0 |
| Silicone oil | (e) (parts by mass) | 0 | 0 | 0 | 0 | 0 | 0 | 0 |
| Sheet characteristics | Amount of grains | ◎ | ○ | ○ | ○ | ○ | Δ | Δ |
| Plug body characteristics | Resealability after steam sterilization treatment | ○ | ○ | Δ | Δ | ○ | Δ | Δ |
| | Needlestick resistance | ○ | Δ | Δ | Δ | ○ | ○ | Δ |
| | Coring resistance | ○ | Δ | Δ | Δ | ○ | Δ | Δ |
| | Odor sensory test | 2 | 2 | 2.5 | 2.5 | 1.5 | 1.5 | 1 |
| | Oil bleed resistance | ○ | ○ | ○ | ◎ | Δ | Δ | Δ |

[Table 10]

| | | Example 37 | Example 38 | Example 39 | Example 40 | Example 41 | Example 42 |
|---|---|---|---|---|---|---|---|
| Hydrogenated block copolymer | (a) Type of polymer | Polymer 1 | Polymer 1 | Polymer 1 | Polymer 1 | Polymer 1 | Polymer 1 |
| | Amount blended (parts by mass) | 100 | 100 | 100 | 100 | 100 | 100 |
| Polypropylene resin | (b) (parts by mass) | 22 | 22 | 27 | 18 | 22 | 22 |
| Polyphenylene ether resin | (f) (parts by mass) | 50 | 60 | 50 | 50 | 60 | 60 |
| Non-aromatic softener | (c-1) (parts by mass) | 50 | 50 | 50 | 50 | 50 | 50 |
| | (c-2) (parts by mass) | 100 | 100 | 100 | 100 | 100 | 100 |
| Inorganic filler | (d-1) (parts by mass) | 25 | 30 | 25 | 25 | 20 | 15 |
| Silicone oil | (e) (parts by mass) | 3 | 3 | 3 | 3 | 0 | 0 |
| Sheet characteristics | Amount of grains | Δ | Δ | ○ | Δ | Δ | Δ |

(continued)

| | | Example 37 | Example 38 | Example 39 | Example 40 | Example 41 | Example 42 |
|---|---|---|---|---|---|---|---|
| Plug body characteristics | Resealability after steam sterilization treatment | ◎ | ◎ | ◎ | ○ | ◎ | ◎ |
| | Needlestick resistance | ○ | ○ | ○ | Δ | Δ | ○ |
| | Coring resistance | ○ | ○ | ○ | ○ | ○ | ○ |
| | Odor sensory test | 2.5 | 2.5 | 2,5 | 2.5 | 2.5 | 2,6 |
| | Oil bleed resistance | ○ | ○ | ○ | ○ | ○ | ○ |

[Table 11]

| | | Comparative Examples 32 | Comparative Examples 33 | Comparative Examples 34 |
|---|---|---|---|---|
| Hydrogenated block copolymer | (a) Type of polymer | Polymer 11 | Polymer 12 | Polymer 24 |
| | Amount blended (parts by mass) | 100 | 100 | 100 |
| Polypropylene resin | (b) (parts by mass) | 50 | 35 | 20 |
| Polyphenylene ether resin | (f) (parts by mass) | 0 | 20 | 20 |
| Non-aromatic softener | (c-1) (parts by mass) | 10 | 50 | 50 |
| | (c-2) (parts by mass) | 20 | 100 | 100 |
| Inorganic filler | (d-1) (parts by mass) | 0 | 20 | 20 |
| Silicone oil | (e) (parts by mass) | 0 | 3 | 3 |
| Sheet characteristics | Amount of grains | ∇ | ○ | ∇ |
| Plug body characteristics | Resealability after steam sterilization treatment | Δ | Δ | ○ |
| | Needlestick resistance | Δ | × | ○ |
| | Coring resistance | ∇ | ∇ | ∇ |
| | Odor sensory test | 0 | 1.5 | 1.5 |
| | Oil bleed resistance | ○ | ○ | × |

Industrial Applicability

The hydrogenated block copolymer of the present invention, which has a low shear melt viscosity and excellent processability as well as has a favorable compression set at 70°C specific to polymers, is expected to be developed in various applications. The hydrogenated block copolymer, which is also highly compatible with polypropylene and of which crystals are miniaturized, can suppress occurrence of gel to thereby suppress decrease in designability and decrease in physical properties due to grains. For example, a plug body for medical containers, comprising the hydrogenated block copolymer of the present invention, has an excellent balance among needlestick resistance, resealability, coring resistance, oil bleed resistance, and the like without addition of a polyphenylene ether resin or an inorganic filler. Thus, the plug body has no occurrence of odor derived from a polyphenylene ether resin, and has industrial applicability as a plug body for various medical containers such as transfusion bags.

Reference Signs List

| | |
|---|---|
| 1 | Plug body |
| 2 | Jig |
| 21 | Screw pitch part |
| 22 | Holder |
| 23 | Lock ring |

## Claims

1. A hydrogenated block copolymer (P) obtained by hydrogenation of a coupled polymer represented by the following formula (1):

   (A-B)n-X (1)

   wherein A represents a polymer block comprising a vinyl aromatic monomer unit as a main component, B represents a polymer block comprising a conjugated diene monomer unit as a main component, n is an integer of 1 or greater, and X represents a residue of a coupling agent or a residue of a polymerization initiator, wherein

   the content of the vinyl aromatic monomer unit in the hydrogenated block copolymer (P) is 10 mass% to 40 mass%,
   the peak top molecular weight of a diblock component corresponding to (A-B) in the formula (1) is 160,000 to 225,000,
   the proportion of a dibranched component corresponding to a case in which n is 2 in the formula (1) is 10 mass% to 30 mass% based on the total hydrogenated block copolymer (P),
   the proportion of a tribranched component corresponding to a case in which n is 3 in the formula (1) is 40 mass% to 70 mass% based on the total hydrogenated block copolymer (P), and
   the ratio M (mass of tribranched component/mass of dibranched component) of the tribranched component to the dibranched component is 2 or more, wherein the content of the vinyl aromatic monomer unit in the hydrogenated block copolymer (P), the peak top molecular weight of a diblock component, the proportion of a dibranched component, the proportion of a tribranched component, the ratio M (mass of tribranched component/mass of dibranched component) of the tribranched component to the dibranched component are all measured according to the methods as disclosed in the description.

2. The hydrogenated block copolymer according to claim 1, wherein the proportion of the diblock component is 10 mass% to 40 mass% based on the total hydrogenated block copolymer (P).

3. The hydrogenated block copolymer according to claim 1 or 2, wherein the proportion of a tetra- or higher-branched component corresponding to a case in which n is an integer of 4 or more in the formula (1) is 10 mass% or less based on the total hydrogenated block copolymer (P), measured as disclosed in the description.

4. The hydrogenated block copolymer according to any one of claims 1 to 3, wherein the weight average molecular weight of the total hydrogenated block copolymer (P) is 350,000 to 500,000 measured as disclosed in the description.

5. The hydrogenated block copolymer according to any one of claims 1 to 4, wherein the proportion of a 1,2-bond and a 3,4-bond in the conjugated diene monomer unit in the hydrogenated block copolymer (P) is 50 mol% to 80 mol%, measured as disclosed in the description.

6. The hydrogenated block copolymer according to any one of claims 1 to 5, wherein the degree of hydrogenation of the conjugated diene monomer unit in the hydrogenated block copolymer (P) is 80 mol% or more, measured as disclosed in the description.

7. An elastomer composition comprising the hydrogenated block copolymer according to any one of claims 1 to 6.

8. The elastomer composition according to claim 7, comprising 10 to 50 parts by mass of a polypropylene resin and

30 to 200 parts by mass of a non-aromatic softener based on 100 parts by mass of the hydrogenated block copolymer.

9. The elastomer composition according to claim 7 or 8, wherein, when an elastomer composition sheet produced under the following conditions is cut into a 10 cm × 10 cm square and both the surfaces are observed with a microscope, the number of grains having a diameter of 200 μm or more calculated by the following expression is 0; [Production of Elastomer Composition Sheet]
a sheet material comprising the hydrogenated block copolymer is melt-kneaded with a twin screw extruder at a setting temperature of 230°C, and the obtained kneaded product is molded using a T die at a number of revolutions of the screws of 300 rpm to produce an elastomer composition sheet having a thickness of 500 μm;

Number of grains = [total number of grains having a diameter of 200 μm or more observed] × Y/X

wherein X represents a weight of the composition sheet (g), Y represents an amount of the hydrogenated block copolymer in the composition sheet (g), and a calculated value rounded off to the nearest whole number is employed as the number of the grains.

10. The elastomer composition according to any one of claims 7 to 9, comprising no polyphenylene ether resin.

11. The elastomer composition according to any one of claims 7 to 10, comprising no surface-treated silica.

12. The elastomer composition according to any one of claims 7 to 9, comprising no polyphenylene ether resin and surface-treated silica.

13. A seal member comprising the elastomer composition according to any one of claims 7 to 12.

14. A plug body comprising the elastomer composition according to any one of claims 7 to 12.

15. A medical plug for use in a medical application, comprising the elastomer composition according to any one of claims 7 to 12.

## Patentansprüche

1. Hydriertes Blockcopolymer (P), erhalten durch Hydrierung eines durch die folgende Formel (1) dargestellten gekuppelten Polymers,

(A-B)n-X (1)

worin A einen Polymerblock darstellt, der eine vinylaromatische Monomereinheit als eine Hauptkomponente umfasst, B einen Polymerblock darstellt, der eine konjugierte Dienmonomereinheit als eine Hauptkomponente umfasst, n eine ganze Zahl von 1 oder größer ist und X einen Rest eines Kupplungsmittels oder einen Rest eines Polymerisationsinitiators darstellt, worin

der Gehalt der vinylaromatischen Monomereinheit im hydrierten Blockcopolymer (P) 10 Massen-% bis 40 Massen-% beträgt,
das Spitzenmolekulargewicht einer Zweiblockkomponente, entsprechend (A-B) in Formel (1), 160000 bis 225000 beträgt,
der Anteil einer zweizweigigen Komponente, entsprechend einem Fall, bei dem n in der Formel (1) 2 ist, 10 Massen-% bis 30 Massen-%, bezogen auf das gesamte hydrierte Blockcopolymer (P), ist,
der Anteil einer dreizweigigen Komponente, entsprechend einem Fall, in dem n in der Formel (1) 3 ist, 40 Massen-% bis 70 Massen-%, bezogen auf das gesamte hydrierte Blockcopolymer (P), ist, und
das Verhältnis M (Masse der dreizweigigen Komponente/Masse der zweizweigigen Komponente) zwischen der dreizweigigen Komponente und der zweizweigigen Komponente 2 oder mehr ist.

2. Hydriertes Blockcopolymer nach Anspruch 1, wobei der Anteil der Zweiblockkomponente 10 bis 40 Massen-%, bezogen auf das gesamte hydrierte Blockcopolymer (P), ist.

3. Hydriertes Blockcopolymer nach Anspruch 1 oder 2, wobei der Anteil einer tetra- oder höherzweigigen Komponente,

entsprechend einem Fall, bei dem n eine ganze Zahl von 4 oder mehr in der Formel (1) ist, 10 Massen-% oder weniger, bezogen auf das gesamte hydrierte Blockcopolymer (P), ist.

4. Hydriertes Blockcopolymer nach einem der Ansprüche 1 bis 3, wobei das gewichtsmittlere Molekulargewicht des gesamten hydrierten Blockcopolymers (P) 350000 bis 500000 beträgt, gemessen wie in der Beschreibung beschrieben.

5. Hydriertes Blockcopolymer nach einem der Ansprüche 1 bis 4, wobei der Anteil an 1,2-Bindung und 3,4-Bindung in der konjugierten Dienmonomereinheit in dem hydrierten Blockcopolymer (P) 50 Mol-% bis 80 Mol-% beträgt, gemessen wie in der Beschreibung beschrieben.

6. Hydriertes Blockcopolymer nach einem der Ansprüche 1 bis 5, wobei der Hydrierungsgrad der konjugierten Dienmonomereinheit in dem hydrierten Blockcopolymer (P) 80 Mol-% oder mehr beträgt, gemessen wie in der Beschreibung beschrieben.

7. Elastomerzusammensetzung, welche das hydrierte Blockcopolymer nach einem der Ansprüche 1 bis 6 enthält.

8. Elastomerzusammensetzung nach Anspruch 7, umfassend 10 bis 50 Massenteile eines Polypropylenharzes und 30 bis 200 Massenteile eines nichtaromatischen Weichmachers, bezogen auf 100 Massenteile des hydrierten Blockcopolymers.

9. Elastomerzusammensetzung nach Anspruch 7 oder 8, wobei, wenn eine unter den folgenden Bedingungen hergestellte Elastomerzusammensetzungsbahn in ein 10 cm× 10 cm großes Quadrat geschnitten wird und beide Oberflächen mit einem Mikroskop beobachtet werden, die Anzahl der Körner mit einem Durchmesser von 200 μm oder mehr, berechnet durch den folgenden Ausdruck, 0 ist; [Herstellung von Elastomerverbundbahn]

   ein Bahnmaterial, welches das hydrierte Blockcopolymer enthält, wird mit einem Doppelschneckenextruder bei einer Erstarrungstemperatur von 230° C schmelzgeknetet, und das erhaltene geknetete Produkt wird unter Verwendung einer T-Düse bei einer Umdrehungszahl der Schnecken von 300 U/min geformt, um eine Elastomerzusammensetzungsbahn mit einer Dicke von 500 μm herzustellen;

   Anzahl der Körner = [Gesamtzahl der beobachteten Körner mit einem Durchmesser von 200 μm oder mehr] × Y/X

   worin X das Gewicht der Zusammensetzungsbahn (g) darstellt, Y die Menge des hydrierten Blockcopolymers in der Zusammensetzungsbahn (g) darstellt und ein berechneter Wert, der auf die nächste ganze Zahl gerundet ist, als die Anzahl der Körner verwendet wird.

10. Elastomerzusammensetzung nach einem der Ansprüche 7 bis 9, welche kein Polyphenylenetherharz enthält.

11. Elastomerzusammensetzung nach einem der Ansprüche 7 bis 10, welche kein oberflächenbehandeltes Siliciumdioxid enthält.

12. Elastomerzusammensetzung nach einem der Ansprüche 7 bis 9, welche kein Polyphenylenetherharz und oberflächenbehandeltes Siliciumdioxid enthält.

13. Dichtungselement, das die Elastomerzusammensetzung nach einem der Ansprüche 7 bis 12 enthält.

14. Stopfenkörper, der die Elastomerzusammensetzung nach einem der Ansprüche 7 bis 12 enthält.

15. Medizinischer Stopfen zur Verwendung in einer medizinischen Anwendung, umfassend die Elastomerzusammensetzung nach einem der Ansprüche 7 bis 12.

## Revendications

1. Copolymère séquencé hydrogéné (P) obtenu par hydrogénation d'un polymère couplé représenté par la formule

suivante (1) :

(A-B)n-X (1)

dans lequel A représente une séquence de polymère comprenant un motif monomère vinylique aromatique en tant que composant principal, B représente une séquence de polymère comprenant un motif monomère diène conjugué en tant que composant principal, n est un nombre entier de 1 ou supérieur, et X représente un résidu d'un agent de couplage ou un résidu d'un initiateur de polymérisation, dans lequel

la teneur du motif monomère vinylique aromatique dans le copolymère séquencé hydrogéné (P) est de 10 % en masse à 40 % en masse,

le poids moléculaire de sommet de pic d'un composant diséquencé correspondant à (A-B) dans la formule (1) est de 160 000 à 225 000,

la proportion d'un composant diramifié correspondant à un cas dans lequel n vaut 2 dans la formule (1) est de 10 % en masse à 30 % en masse sur la base du copolymère séquencé hydrogéné (P) total,

la proportion d'un composant triramifié correspondant à un cas dans lequel n vaut 3 dans la formule (1) est de 40 % en masse à 70 % en masse sur la base du copolymère séquencé hydrogéné (P) total, et

le rapport M (masse de composant triramifié/masse de composant diramifié) du composant triramifié au composant diramifié est de 2 ou plus, dans lequel la teneur du motif monomère vinylique aromatique dans le copolymère séquencé hydrogéné (P), le poids moléculaire de sommet de pic d'un composant diséquencé, la proportion d'un composant diramifié, la proportion d'un composant triramifié, le rapport M (masse de composant triramifié/masse de composant diramifié) du composant triramifié au composant diramifié sont tous mesurés selon les procédés tels que divulgués dans la description.

**2.** Copolymère séquencé hydrogéné selon la revendication 1, dans lequel la proportion du composant diséquencé est de 10 % en masse à 40 % en masse sur la base du copolymère séquencé hydrogéné (P) total.

**3.** Copolymère séquencé hydrogéné selon la revendication 1 ou 2, dans lequel la proportion d'un composant tétraramifié ou comportant davantage de ramifications correspondant à un cas dans lequel n est un nombre entier de 4 ou plus dans la formule (1) est de 10 % en masse ou moins sur la base du copolymère séquencé hydrogéné (P) total, mesurée tel que divulgué dans la description.

**4.** Copolymère séquencé hydrogéné selon l'une quelconque des revendications 1 à 3, dans lequel le poids moléculaire moyen en poids du copolymère séquencé hydrogéné (P) total est de 350 000 à 500 000, mesuré tel que divulgué dans la description.

**5.** Copolymère séquencé hydrogéné selon l'une quelconque des revendications 1 à 4, dans lequel la proportion d'une liaison 1,2 et d'une liaison 3,4 dans le motif monomère diène conjugué dans le copolymère séquencé hydrogéné (P) est de 50 % en moles à 80 % en moles, mesurée tel que divulgué dans la description.

**6.** Copolymère séquencé hydrogéné selon l'une quelconque des revendications 1 à 5, dans lequel le degré d'hydrogénation du motif monomère diène conjugué dans le copolymère séquencé hydrogéné (P) est de 80 % en moles ou plus, mesuré tel que divulgué dans la description.

**7.** Composition d'élastomère comprenant le copolymère séquencé hydrogéné selon l'une quelconque des revendications 1 à 6.

**8.** Composition d'élastomère selon la revendication 7, comprenant 10 à 50 parties en masse d'une résine de polypropylène et 30 à 200 parties en masse d'un plastifiant non aromatique sur la base de 100 parties en masse du copolymère séquencé hydrogéné.

**9.** Composition d'élastomère selon la revendication 7 ou 8, dans laquelle, lorsqu'une feuille de composition d'élastomère produite dans les conditions suivantes est découpée en un carré de 10 cm × 10 cm et que les deux surfaces sont observées avec un microscope, le nombre de grains présentant un diamètre de 200 μm ou plus calculé par l'expression suivante est de 0 ;
[Production de feuille de composition d'élastomère]

un matériau de feuille comprenant le copolymère séquencé hydrogéné est malaxé à l'état fondu avec une extrudeuse à double vis à une température de réglage de 230 °C, et le produit malaxé obtenu est moulé en

utilisant une matrice en T à un nombre de révolutions des vis de 300 révolutions par minute pour produire une feuille de composition d'élastomère présentant une épaisseur de 500 $\mu$m ;

Nombre de grains = [nombre total de grains présentant un diamètre de 200 $\mu$m ou plus observés] $\times$ Y/X

dans lequel X représente un poids de la feuille de composition (g), Y représente une quantité du copolymère séquencé hydrogéné dans la feuille de composition (g), et une valeur calculée arrondie au nombre entier le plus proche est employée en tant que nombre des grains.

**10.** Composition d'élastomère selon l'une quelconque des revendications 7 à 9, ne comprenant pas de résine d'éther de polyphénylène.

**11.** Composition d'élastomère selon l'une quelconque des revendications 7 à 10, ne comprenant pas de silice traitée en surface.

**12.** Composition d'élastomère selon l'une quelconque des revendications 7 à 9, ne comprenant pas de résine d'éther de polyphénylène et de silice traitée en surface.

**13.** Élément d'étanchéité comprenant la composition d'élastomère selon l'une quelconque des revendications 7 à 12.

**14.** Corps de bouchon comprenant la composition d'élastomère selon l'une quelconque des revendications 7 à 12.

**15.** Bouchon médical pour une utilisation dans une application médicale, comprenant la composition d'élastomère selon l'une quelconque des revendications 7 à 12.

[FIG. 1]

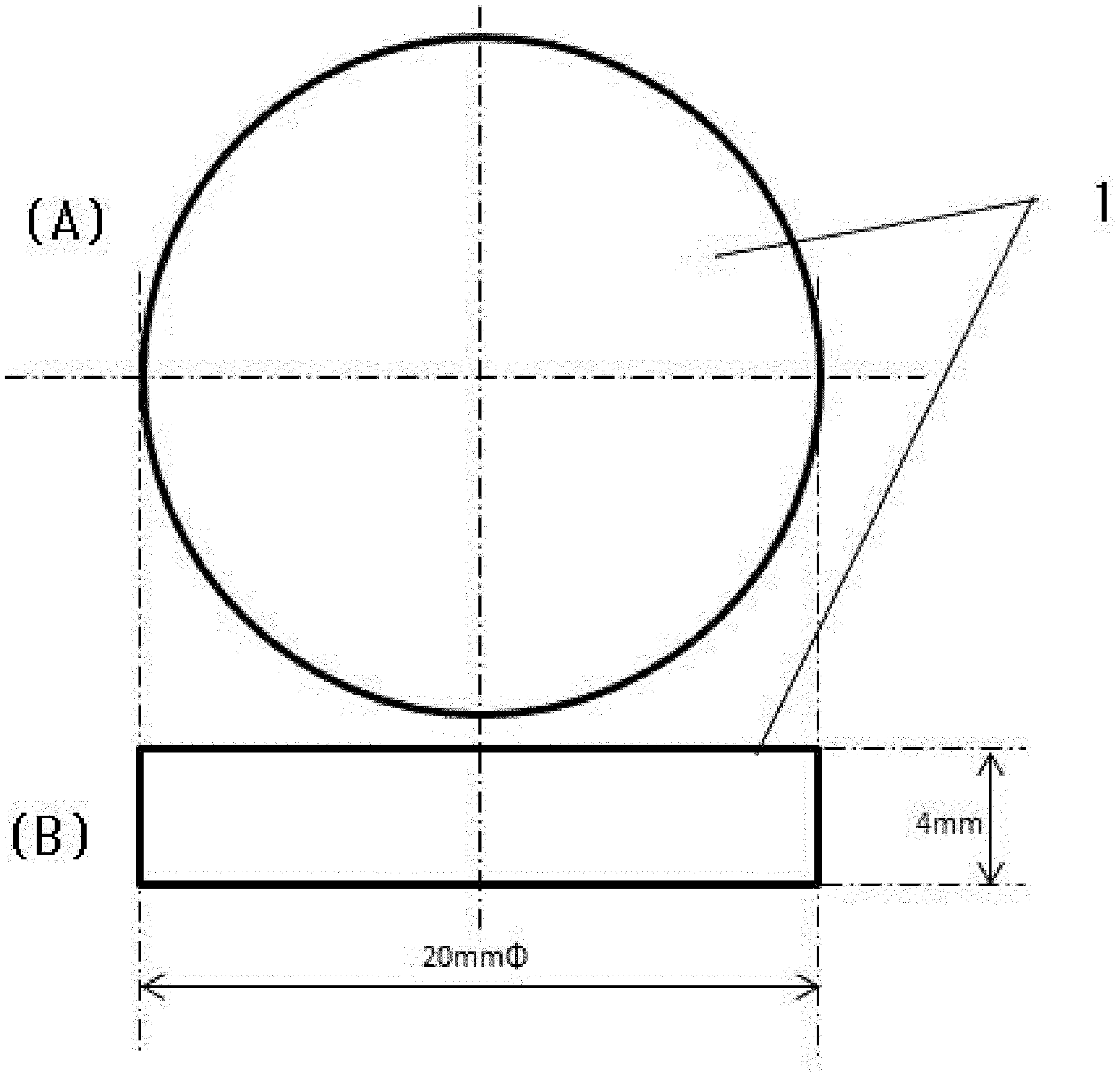

[FIG. 2]

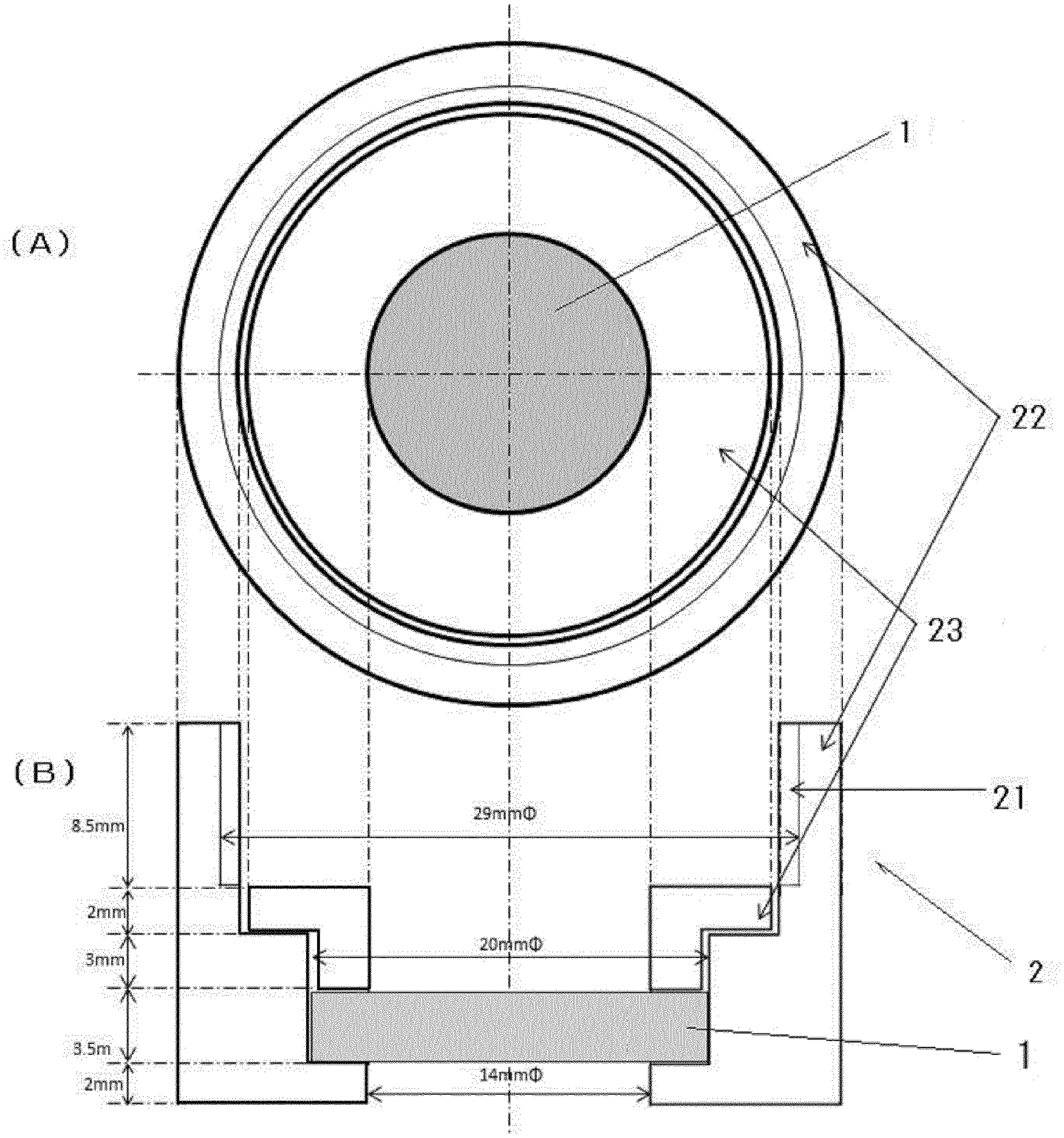

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5591346 B
- JP 2764746 B
- JP 2001240720 A
- JP 3378582 B
- JP 5324426 B
- JP 5703990 B
- WO 2018139122 A
- JP 2020019947 A
- US 3306874 A
- US 3306875 A
- US 3257357 A
- US 3257358 A
- JP 50051197 A
- JP 52017880 A
- JP 63152628 A